# EUROPEAN PATENT SPECIFICATION

(11) **EP 3 469 246 B1**
(45) Date of publication and mention of the grant of the patent: **09.02.2022**
(21) Application number: 17813822.8
(22) Date of filing: 08.06.2017
(51) Int. Cl.: F16L 37/098, A61M 39/10, F16L 37/00, F16L 37/04, F16L 37/084, F16L 37/133

(54) **FLUIDIC CONNECTOR ASSEMBLY FOR QUICK CONNECT/ DISCONNECT**
FLUIDVERBINDERANORDNUNG FÜR SCHNELLVERBINDUNG/SCHNELLLÖSUNG
ENSEMBLE DE CONNECTEUR FLUIDIQUE POUR CONNEXION/DÉCONNEXION RAPIDE

(30) Priority: 13.06.2016 US 201662349569 P
(43) Date of publication of application: 17.04.2019
(73) Proprietor: Idex Health & Science LLC, Oak Harbor, WA 98277 (US)
(72) Inventor: BEEMER, Eric, Anacortes, WA 98221 (US); ELLIS, Scott, Anacortes, WA 98221 (US); GRAHAM, Craig, Anacortes, WA 98221 (US); NIENHUIS, Nathaniel, Coupevilie, WA 98239 (US); SANDERS, Troy, Oak Harbor, WA 98277 (US)
(74) Representative: Jones, Nicholas Andrew
(86) International application number: PCT/US2017/036590
(87) International publication number: WO 2017/218294

(56) References cited:
- US-A- 2 748 804
- US-A- 2 748 804
- US-A- 3 245 703
- US-A- 3 245 703
- US-A- 4 991 883
- US-A- 4 991 883
- US-A1- 2002 093 194
- US-A1- 2002 101 079
- US-A1- 2005 057 042
- US-A1- 2007 052 237
- US-A1- 2007 205 567
- US-A1- 2011 006 519

## Description

### FIELD OF THE INVENTION

This invention relates generally to fluidic connections and fitting assemblies, and more specifically to fluidic connections and fittings which are easy to use, provide reliable sealing connections, and can be used without additional tools or equipment, and more particularly to fluidic connections and fitting assemblies for making fluidic connections or disconnecting fluidic connections quickly, such as may be useful in analytical instrument systems.

### BACKGROUND OF THE INVENTION

There are numerous types of conventional analytical instrument (AI) systems in use and commercially available. Conventional AI systems include systems for Liquid chromatography (LC), ion chromatography (IC), and gas chromatography (GC). In addition, AI systems include high-pressure liquid chromatography, ultra-high pressure liquid chromatography, mass spectrometry systems, micro-flow chromatography systems, nanoflow and nano-scale chromatography systems, capillary electrophoresis systems, reverse-gradient chromatography systems, and systems which include or combine one or more of the foregoing. Although the following background discussion focuses on liquid chromatography systems and applications, those skilled in the art that the references to LC systems and applications are exemplary only and for the convenience of the reader, and are not limiting in any fashion.

LC systems provide well-known techniques for separating the constituent elements in a given sample. In a conventional LC system, a liquid solvent (referred to as the "mobile phase") is introduced from a reservoir and is pumped through the LC system. The mobile phase exits the pump under pressure. The mobile phase then travels via tubing to a sample injection valve. As the name suggests, the sample injection valve allows an operator to inject a sample into the LC system, where the sample will be carried along with the mobile phase.

In a conventional LC system, the sample and mobile phase pass through one or more filters and often a guard column before coming to the column. A typical column usually consists of a piece of tubing which has been packed with a "packing" material. The "packing" consists of the particulate material "packed" inside the column. It usually consists of silica- or polymer-based particles, which are often chemically bonded with a chemical functionality. When the sample is carried through the column (along with the mobile phase), the various components in the sample migrate through the packing within the column at different rates (*i.e.,* there is differential migration of the solutes). In other words, the various components in a sample will move through the column at different rates. Because of the different rates of movement, the components gradually separate as they move through the column. Differential migration is affected by factors such as the composition of the mobile phase, the composition of the stationary phase (*i.e.,* the material with which the column is "packed"), and the temperature at which the separation takes place. Thus, such factors will influence the separation of the sample's various components.

Once the sample (with its components now separated) leaves the column, it flows with the mobile phase past a detector. The detector detects the presence of specific molecules or compounds. Two general types of detectors are used in LC applications. One type measures a change in some overall physical property of the mobile phase and the sample (such as their refractive index). The other type measures only some property of the sample (such as the absorption of ultraviolet radiation). In essence, a typical detector in a LC system can measure and provide an output in terms of mass per unit of volume (such as grams per milliliter) or mass per unit of time (such as grams per second) of the sample's components. From such an output signal, a "chromatogram" can be provided; the chromatogram can then be used by an operator to determine the chemical components present in the sample. Additionally, LC systems may utilize mass spectrometric detection for identification and quantification of the sample, either in addition to, or as an alternative to, the conventional detectors described previously. Ion chromatography relies on the detection of ions in solution, so most metallic materials in the flow path can create interference in the detection scheme, as they create background ions.

In addition to the above components, a LC system will often include filters, check valves, a guard column, or the like in order to prevent contamination of the sample or damage to the LC system. For example, an inlet solvent filter may be used to filter out particles from the solvent (or mobile phase) before it reaches the pump. A guard column is often placed before the analytical or preparative column; *i.e.,* the primary column. The purpose of such a guard column is to "guard" the primary column by absorbing unwanted sample components that might otherwise bind irreversibly to the analytical or preparative column.

In practice, various components in an LC system may be connected by an operator to perform a given task. For example, an operator will select an appropriate mobile phase and column, and then connect a supply of the selected mobile phase and a selected column to the LC system before operation. In order to be suitable for LC applications, each connection must be able to withstand the typical operating pressures of the LC system. If the connection is too weak, it may leak. Because the types of solvents that are sometimes used as the mobile phase are often toxic and because it is often expensive to obtain and/or prepare many samples for use, any such connection failure is a serious concern.

It is fairly common for an operator to disconnect a column (or other component) from a LC system and then connect a different column (or other component) in its place after one test has finished and before the next begins. Given the importance of leak-proof connections in LC applications, the operator must take time to be sure the connection is sufficient. Replacing a column (or other component) may occur several times in a day. Moreover, the time involved in disconnecting and then connecting a column (or other component) is unproductive because the LC system is not in use and the operator is engaged in plumbing the system instead of preparing samples or other more productive activities. Hence, the replacement of a column (or other component) in a conventional LC system can involve a great deal of wasted time and inefficiencies.

Given concerns about the need for leak-free connections, conventional connections have been made with stainless steel tubing and stainless steel end fittings. More recently, however, it has been realized that the use of stainless steel components in a LC system have potential drawbacks in situations involving biological samples, and cannot be routinely used for ion chromatography. For example, the components in a sample may attach themselves to the wall of stainless steel tubing. This presents problems because the detector's measurements (and thus the chromatogram) of a given sample may not accurately reflect the sample if some of the sample's components or ions remain in the tubing and do not pass the detector. Perhaps of even greater concern, however, is the fact that ions from the stainless steel tubing may detach from the tubing and flow past the detector, thus leading to potentially erroneous results. Hence, there is a need for "biocompatible" or "metal-free" connections through the use of a material that is chemically inert with respect to such "biological" samples and the mobile phase used with such samples, so that ions will not be released by the tubing and thus contaminate the sample.

In many applications using selector/injector valves to direct fluid flows, and in particular in liquid chromatography, the volume of fluids is small. This is particularly true when liquid chromatography is being used as an analytical method as opposed to a preparative method. Such methods often use capillary columns and are generally referred to as capillary chromatography. In capillary chromatography, it is often desired to minimize the internal volume of the selector or injector valve. One reason for this is that a valve having a large volume will contain a relatively large volume of liquid, and when a sample is injected into the valve the sample will be diluted, decreasing the resolution and sensitivity of the analytical method.

Micro-fluidic analytical processes also involve small sample sizes. As used herein, sample volumes considered to involve micro-fluidic techniques can range from as low as volumes of only several picoliters or so, up to volumes of several milliliters or so, whereas more traditional LC techniques, for example, historically often involved samples of about one microliter to about 100 milliliters in volume. Thus, the micro-fluidic techniques described herein involve volumes one or more orders of magnitude smaller in size than traditional LC techniques. Micro-fluidic techniques can also be expressed as those involving fluid flow rates of about 0.5 ml/minute or less.

As noted, liquid chromatography (as well as other analytical) systems typically include several components. For example, such a system may include a pump; an injection valve or autosampler for injecting the analyte; a precolumn filter to remove particulate matter in the analyte solution that might clog the column; a packed bed to retain irreversibly adsorbed chemical material; the LC column itself; and a detector that analyzes the carrier fluid as it leaves the column. Ion chromatography may also utilize a suppressor column to facilitate detection dynamic range. These various components may typically be connected by a miniature fluid conduit, or tubing, such as metallic or polymeric tubing (for ion chromatography), usually having an internal diameter of 0.003 to 0.040 inch.

All of these various components and lengths of tubing are typically interconnected by threaded fittings. Fittings for connecting various LC system components and lengths of tubing are disclosed in prior patents, for example, U.S. Patent Nos. 5,525,303; 5,730,943; and 6,095,572, the disclosures of which are herein all incorporated by reference as if fully set forth herein. Often, a first internally threaded fitting seals to a first component with a ferrule or similar sealing device. The first fitting is threadedly connected through multiple turns by hand or by use of a wrench or wrenches to a second fitting having a corresponding external fitting, which is in turn sealed to a second component by a ferrule or other seal. Disconnecting these fittings for component replacement, maintenance, or reconfiguration often requires the use of a wrench or wrenches to unthread the fittings. Although a wrench or wrenches may be used, other tools such as pliers or other gripping and holding tools are sometimes used. In addition, the use of such approaches to connect components of an LC system often results in deformation or swaging of a ferrule used to provide a leak proof seal of tubing to a fitting or component. This often means that the ferrule and tubing connection, once made, cannot be reused without a risk of introducing dead volumes into the system. In addition, such approaches may involve crushing or deformation of the inner diameter of the tubing, which may adversely affect the flow characteristics and the pressures of the fluid within the tubing.

Another approach to provide a connection in an LC system involves providing a fitting assembly that uses a combination of components, including two separate ferrules. Such an approach is considered undesirable because by requiring two places for the ferrules to provide leak proof seals, it provides two places where the fluid to be analyzed may leak, as well as where dead volumes may be provided. In addition, this approach involves the use of additional components, which can cost more and also increase the time and effect to assemble them to make a connection or disassemble them when disconnecting tubing from a component or other fitting assembly.

There exists a need for fluidic fittings that are more reliable and have increased performance, which can be accomplished by applying a specific amount of torque to a fluidic fitting. The long used standard of "finger tight" when applying torque introduces a great deal of variation into the process. This results in fittings being under tightened, which causes leaks, or potentially over-tightened (with a tool), which can result in damage to fittings and ports. In general a torque limiting fitting may be preferred over the use a torque tool (such as a torque wrench) since torque tools require specific designs to allow access to specific fittings, employee training, additional assembly time, and associated costs (e.g., tool purchase and periodic calibration).

U.S. Patent No. 5,183,140 discloses a general torque limiting mechanism, which comprises two rotatable members, one of which is the driving member and the other of which is the driven member. One of the members includes a single radial projection extending from a central hub that engages a recessed area on the other member. Below the torque limit the projection engages the recessed area and allows the driving member to drive the driven member, but above the torque limit the projection disengages the recessed area and prohibits the driving member from driving the driven member. U.S. Patent 7,984,933 discloses a torque limiting fitting, which also comprises two rotatable members, one of which is the driving member and the other of which is the driven member. One of the members includes a lever extending from a central hub that engages an abutment on the other member. Below the torque limit the lever engages the abutment and allows the driving member to drive the driven member, but above the torque limit the lever deflects from the abutment and prohibits the driving member from driving the driven member. However, the radial projection and the lever are only supported on one end, which can result in inconsistency in the torque limit and generally lower maximum torque values.

It will be understood by those skilled in the art that, as used herein, the term "LC system" is intended in its broad sense to include all apparatus and components in a system used in connection with a liquid chromatography system, and that the discussion of fittings in the context of LC systems is exemplary, as the invention may apply beyond LC systems to gas and ion chromatography, as well as or in vitro diagnostic or environmental analysis, and in other analytical instruments and systems, and may be made of only a few simple components or made of numerous, sophisticated components which are computer controlled or the like. Those skilled in the art will also appreciate that an LC system is one type of an analytical instrument (AI) system. Those skilled in the art will appreciate that much of the foregoing discussion with respect to LC systems also has application to other types of AI systems and methods.

Conventional fitting assemblies in certain applications will use fittings that are screwed into ports. Such conventional fittings do not include an energizing member to supply a constant force, which may be needed if the fitting assembly or one or more of its components changes (e.g., such as via creep) over time.

Experience teaches that most users of equipment involving such fluidic connections do not like using tools to install fittings. Most prefer to make fluidic connections by hand and to disconnect the fluidic connections, once made, by hand. Moreover, most users do not like to be required to apply torque to threaded fittings (whether by hand or by use of a tool) to a specific torque value or range or values, often due to the time or extra equipment needed to do so. Also, in many situations, it is not entirely clear if an operator has applied enough or too much torque in making up a connection until the AI system is in operation and a leak occurs or the pressure of the fluid flowing through a tube causes its extrusion from the port.

In many conventional fluidic connections, it can be difficult to determine if the fitting assembly is correctly or fully installed or not, unless and until a leak or burst occurs. For example, a fitting assembly may appear fine because it is partially screwed into a threaded port, but in fact is not fully engaged to provide a seal at a desired pressure range for a given application because the user failed to apply sufficient torque when making the connection. Conventional fluidic connections and fitting assemblies do not provide an easily observable indication of whether the fitting assembly is fully connected.

Attempts have been made to try to address some of the drawbacks and issues with conventional fitting assemblies and connections. For example, attempts have been made to develop approaches intended to provide fittings which may be quickly connected to and disconnected from a component. In U.S. Patent No. 5,803,512, titled "Tube Quick Connect to Female Socket," issued to Hollnagel on September 8, 1998, a quick connect assembly is described, which relies on a pair of slanted arms, the radial deflection of which allows a fitting to connect and disconnect with a female socket member. Likewise, U.S. Patent No. 4,834,423, titled "Quick Connect Fluid Fitting Assembly," issued to DeLand on May 30, 1989, describes a quick-connect fitting assembly which relies on a retainer means including at least one finger which can connect and release in interlocking engagement with a socket. Similarly, U.S. Patent No. 8,448,994, titled "Latch Assembly For Joining Two Conduits," issued to Pisula et al. on May 28, 2013, describes a latch assembly for the connection of conduits, where the connection is established by engagement of a feature within a slot and is disengaged by a release button. U.S. Patent No. 6,497,433, titled "Coupling Assemblies for Providing Fluid Connection," issued to Ketcham on December 4, 2002, also discloses a retainer feature, in this case relying on at least two locking beams to engage and release a conduit to and from a female connector body.

Other attempts have been made to provide various connection assemblies. For example, U.S. Patent No. 4,781,399, titled "Quick-Connect Coupling Having Improved Seal" and issued on November 1, 1988, describes a connection assembly with a ball detent and seal for a household faucet or the like. U.S. Patent No. 4,135,745, titled "Quick Connect Fluid Fitting" and issued on January 23, 1979, describes a connection assembly with an expandable clip on a tube intended to allow tubing to be attached to a housing with a preassembled nut. U.S. published patent application No. 2014/0102561, published on April 17, 2014 and titled "Quick-Release Connectors and Connection Assemblies for Fluidic Coupling" describes the use of a plunger body for use with a check valve. U.S. Patent No. 7,695,020, issued on April 13, 2010, and titled "Coupling with Latch Mechanism," describes a coupling apparatus with a mechanical latch assembly with a latch plate. U.S. Patent No. 6,361,687, titled "Quarter Turn Quick Connect Fitting" and issued on March 26, 2002, describes a fitting assembly with a quarter turn nut which cooperates with a fitting with pins. U.S. Patent No. 6,149,127, issued on November 21, 2000, and titled "Spring Loaded Compression Valve Fitting" describes a valve fitting to be coupled to a valve body and cause a compression member to limit the pressure exerted on the valve body. U.S. Patent No. 5,951,063, titled "Quick Connector with Snap-On Retainer Having Enhanced Engagement" and issued on September 14, 1999, describes a retainer with lock projections for releasably locking together male and female components. It is believed that none of these prior attempts have provided a connection assembly which can quickly and easily be used by an operator to connect or disconnect tubing without the need for any torque or any rotation of a component of the assembly, and without any tools, and yet provide a sealing engagement for high pressure applications in a cost effective manner. U.S. Patent Nos. 4,781,399, 4,135,745, 7,695,020, 6,361,687, 6,149,127, and 5,951,063, and U.S. published patent application No. 2014/0102561.

None of these references disclose a means for supplying constant axial force on the fitting as the fitting changes over time (e.g., the fitting material creeps). Such a force can be advantageous to maintain the integrity of the tube-to-port seal as the fitting assembly creeps. If the fitting assembly creeps and there is no compensating axial force, this could result in leakage at the point of the tube-to-port interface and could thus compromise the fitting assembly. In addition, none of the references noted above disclose a tube-to-port seal wherein the seal is generated solely by the tube face, without the need for torque or extra components (e.g., ferrules). Moreover, conventional fitting assemblies fail to provide a connection system in which an operator can easily determine from visual examination that a seal has been obtained when a connection has been made. US 3,245,703 A discloses a fluidic connection assembly wherein latch portions are adapted to be retained in a port when inserted therein, while a body has a passageway there-through and has a plurality of hinges, each of the hinges corresponding to one of the sides of said body. Further pertinent prior art is disclosed in US 4,991,883 A.

### SUMMARY OF THE INVENTION

In one embodiment of the present disclosure, a fluidic connection assembly is provided which includes a body having a first end and a second end, and having a plurality of sides, and further having a bottom portion with a plurality of latch portions, each corresponding to one of the sides of the body, wherein the latch portions are adapted to be retained in a port when inserted therein, said body further having a passageway therethrough adapted to receive a tube extending through said body, said body further having a plurality of hinges, each of the hinges corresponding to one of the sides of said body, wherein the sides and the corresponding latch portions of said body are adapted to move inwardly towards the longitudinal axis of the body when a force is exerted thereon, thereby allowing the body to be disconnected from the port, and wherein the first end of said body comprises an extension member and the second end of said body is adapted to cooperate with a spring for exerting a force against a bottom of the tube. The fluidic connection assembly can further comprise a second spring in an interior portion of said body such that the second spring exerts a force against the plurality of sides of said body towards the exterior of said body and away from the longitudinal axis of the body. The extension and said body can be integral or can be formed from separate pieces. The second spring can be one or more of any of a number of different types of springs, such as a band spring, a cantilever spring, an elastomeric spring, or the like.

In some embodiments of the present disclosure, the fluidic connection assembly can also include a spring and a tube extending through the extension member, through the body, and through the spring, wherein the spring is located between the second end of said body and one end of said tube. In addition, the end of the tube can comprise a flange and/or the spring can comprise a coiled spring. In some embodiments, the extension portion may include a plurality of toroidal members and/or a spine member, and the spine member can be provided by a wire having a polymeric coating. Some embodiments may further include a backup ring located between the spring and the flange of said tube, wherein the backup ring may be used to help concentrate the force from said spring on the flange in an area smaller than the total surface area of the flange. The backup ring can be radially tapered to concentrate axial force on a smaller area of the tube second end, and the end of the tube may comprise a reduced surface area. The fluidic connection assembly in accordance with the present disclosure may have a body which comprises any one or more of various materials, which may include metal, such as steel, including stainless steel, aluminum, titanium, as well as polymeric materials such as polyetheretherketone (PEEK), polyoxymethylene (POM) such as available under the mark DELRIN and known sometimes as acetal, RADEL brand polyphenylsulfone (PPSU), ULTEM brand polyetherimide (PEI), polyetherketoneketone (PEKK), polyaryletherketone (PAEK), polyethylene, polypropylene, polyvinylchloride, acrylic, and/or other materials, such a fused silica, silica borite, PEEKsil, and the like.

In some embodiments of the present disclosure, methods are provided, including a method of making a connection which includes the steps of providing a tube having first and second ends, wherein the second end comprises a flange, inserting the first end of the tube through a backup ring, a spring, and a body, wherein said body comprises a first end and a second end, a plurality of sides, and a bottom portion having a plurality of latch portions, each corresponding to one of the sides, wherein the latch portions are adapted to be retained in a port when inserted therein, said body further having a passageway therethrough adapted to receive a tube extending through said body, said body further having a plurality of hinges, each of the hinges corresponding to one of the sides of said body, wherein the sides and the corresponding latch portions of said body are adapted to move inwardly towards the center of the body when a force is exerted thereon, and wherein the second end of said body is adapted to cooperate with a spring for exerting a force against a bottom of the tube, and inserting a least a portion of the body, including at least the latch portions of the body, into a port, wherein the latch portions are retained within the port. In at least some embodiments, such methods do not require the application of any torque, or the use of a threaded engagement of two members, or the relative rotation of two threaded members to provide a threaded engagement.

In some embodiments, methods of disconnecting a tube connected to a port are provided. Such methods may include the steps of exerting a force on a plurality of sides of a body in a fluidic connection assembly, wherein the fluidic connection comprises a tube having first and second ends, wherein the second end comprises a flange, and wherein at least a portion of the tube between the second end and the first end extends through the body, wherein the body has a first end and a second end, and a bottom portion with a plurality of latch portions, each corresponding to one of the sides, said body further having a passageway therethrough adapted to receive the tube, said body further having a plurality of hinges, each of the hinges corresponding to one of the sides of said body, wherein the sides and the corresponding latch portions of said body are adapted to move inwardly towards the center of the body when a force is exerted thereon, thereby moving the latch portions of the body inwardly, and then pulling the assembly from the port. In some embodiments of such methods, the steps do not require the application of any torque to a component of the assembly.

In some embodiments of the present disclosure, an analytical instrument system is provided which includes a fluidic connection which in turn includes a body having a first end and a second end, and having a plurality of sides, and further having a bottom portion with a plurality of latch portions, each corresponding to one of the sides, wherein the latch portions are adapted to be retained in a port when inserted therein, said body further having a passageway therethrough adapted to receive a tube extending through said body, said body further having a plurality of hinges, each of the hinges corresponding to one of the sides of said body, wherein the sides and the corresponding latch portions of said body are adapted to move inwardly towards the center of the body when a force is exerted thereon, thereby allowing the body to be disconnected from the port, and wherein the first end of said body comprises an extension member and the second end of said body is adapted to cooperate with a spring for exerting a force against a bottom of the tube. The AI system may further include a spring and a tube extending through the extension member, through the body, and through the spring, wherein the spring is between the second end of said body and one end of said tube. In addition, the spring may exert a force against the flange of the one end of said tube and maintain a sealing engagement. In some embodiments, the AI system may be used for in vitro applications. In at least some of the embodiments of the present disclosure, the fitting assembly, once connected, may provide a seal at a fluid pressure of at least 200 psi, 500 psi, 1000 psi, 2500 psi, 5000 psi, 10000 psi, and 20000 psi. In addition, in some embodiments, the fitting assembly can provide a visual cue to an operator to indicate that a proper connection has been made for an intended application. Moreover, the present disclosure provides apparatus and methods which allow an operator to make up a fluidic connection, or to disconnect a fluidic connection, but hand and without the need for pliers, wrenches, or other tools, and without the need for application of a torque, such as is the case when a connection is made which requires the relative rotation of two threaded members. The connection assembly can be used in connection with any one of a number of ports and/or components in any one or more AI system, including in connection with components including any one or more of the following: a pump, a column, a filter, a guard column, a valve, a detector, a pressure regulator, a reservoir, a degasser, a debubbler, a union, a tee, a cross, an adapter, a splitter, a sample loop, or a connector.

In certain embodiments in accordance with the present disclosure, an assembly body for connecting a tube to a port comprising a body is provided, which has a passageway therethrough and has located therein means for exerting an axial force to bias an end of a tube towards a face of a port, and further has means for removably and securely attaching said body to the port, wherein the means for removably and securely attaching said body are adapted to allow a user to select between a first position and second position of the means for removably and securely attaching said body to thereby attach said body to the port in the first position and to disengage said body from the port in the second position. The end of the tube may further comprise a tip, and the port may be formed by a recess formed in an adapter attached to another component, such as an adapter connected to the end of a column, or may be a port which is part of a manifold, a pump, a valve, a column, a filter, a guard column, a detector, a pressure regulator, a reservoir, a degasser, a debubbler, a union, a tee, a cross, a splitter, a sample loop, a connector, or another component in an AI instrument or system. The means for exerting an axial force may comprises a spring, including without limitation a coiled spring. The means for removably and securely attaching may comprise radial projections on said body adapted to engage with portions of the port, such as radial projections shoulders which latch together when connected, or may comprise a ball detent mechanism. The body is adapted to removably and securely attach to a port with axial loading, such as by an operator by hand or by automated means, without any torque being applied to said body or the port, and without requiring that the body or the port be turned. The tube, the body, and the port assembly may comprise biocompatible materials. In addition, the port may comprise a first inner diameter at an opening thereof and a bottom surface with a reduced surface area for contact with the end of the tube, wherein the bottom surface has an outer diameter less than the first inner diameter of the port. The portion of the tube located within the body may have a first diameter and the end of the tube may comprise a bottom surface having an outer diameter less than the first outer diameter of the tube, thereby concentrating the force exerted at the sealing location and allowing a seal to be formed capable of a leak-free seal and preventing extrusion of the tube from the port without requiring such force to be applied to connect the assembly together by an operator. In addition, the tube may have a tip which has a first end with a first outer diameter and the tip also has a portion thereof with a second outer diameter which is greater than the first outer diameter. The port may be comprised of a combination of two or more distinct components, such as if the port comprises a bottom surface of a first component and an recess extending from the bottom surface is provided by a second component.

In certain embodiments, a fluidic connection assembly for high pressure applications is provided which comprises a connector assembly having a connector and having means for allowing an operator to select between a first position of said connecter and a second position of said connector, wherein in the first position said connector is adapted to be inserted into or removed from a port, and in the second position said connector is adapted to be retained in the port, and wherein said connector has a passageway therethrough adapted to receive a portion of a tube, and means located within said connecter for exerting a force on a portion of tube located within the passageway of said connector when said connector is in the second position, wherein said connector is adapted to be connected to the port without any torque and without turning either said connector or the port, and wherein said connecter assembly and said means for exerting a force on a portion of a tube are adapted to sealingly engage the tube in the port when a fluid flows through the tube. The means for allowing an operator to select between a first position and a second position may comprise a plurality of tabs each connected to a corresponding radial projection which moves radially inward or outward when the tabs are moved radially inward or outward, and such movement may correspond to each other (i.e., inward movement of the projections when the tabs move inward), or may be different from one another (i.e., inward movement of the projections when the tabs are moved outwardly). The means for allowing an operator to select between a first position and a second position may comprise a collar and ball detent. The means for exerting a force may comprises a spring, and the means may be preselected so that the force adapted to be exerted is a force of at least a preselected amount, or within a preselected range. The means for exerting a force and said connector assembly may be adapted to sealingly engage one end of the tube when a fluid flows through the tube at pressures up to 5,000 psi, 10,000 psi, 15,000 psi, 20,000 psi, and/or 25,000 psi.

In certain embodiments of the present disclosure, a fluidic connection assembly is provided and which comprises a body having a first end and a second end, and having a plurality of sides, and further having a bottom portion with a plurality of latch portions, each corresponding to one of the sides, wherein the latch portions are adapted to be retained in a port when inserted therein, said body further having a passageway therethrough adapted to receive a tube extending through said body, said body further having a plurality of sides each having a slot therein, each slot corresponding to one of the sides of said body, wherein each side of said body which has a slot does not have a corresponding latch portion, wherein the sides with corresponding latch portions of said body are adapted to move inwardly towards the center of the body when a force is exerted thereon, thereby allowing said body to be disconnected from the port, and wherein the second end of said body is adapted to cooperate with a spring for exerting a force against a bottom of the tube.

In addition, certain embodiments provide a method of connecting a tube to a port of a component of an analytical instrument system, which method comprises providing an assembly having a first end and a second end, wherein the assembly is adapted to connect to a port of a component of an analytical instrument system, providing a tube having a first end and a second end and extending through the assembly, and inserting, without any threaded engagement of the assembly with the port, at least a portion of the second end of the tube and the second end of the assembly into the port so that a portion of the assembly is removably held in the port and provides a sealed connection between the second end of the tube and the port, wherein the sealed connection is leakproof at pressures of up to at least 2,500 psi.

In other embodiments, a fluidic connection assembly is provided which comprises a body having a first end and a second end, and having a plurality of tabs located between the first and second ends, wherein each of the plurality of tabs extends radially outwardly from said body, and wherein the second end of said body further has a plurality of radially inwardly extending latch portions, each corresponding to one of the tabs, wherein the latch portions are adapted to engage and retain a first end of a port body when the first end of the port body is between the latch portions of said body and the latch portions are in a closed position, and the latch portions are adapted to move radially outwardly when the corresponding tabs move radially inwardly, said body further having a passageway therethrough adapted to receive a tube extending through said body, and a spring located in said body and having a first end proximal the first end of said body and a second end adapted to exert a force to urge one end of the tube against at least a portion of a port in the port body. The fluidic connection assembly may further comprise a spring and a tube extending through the extension member, through the body, and through the spring. The end of said tube may further comprise a PEEK tip, and the spring may comprise a coiled spring. In addition, the assembly may further comprise a boss in the port of the port body which has a diameter smaller than the outer diameter of the tube. The port may comprise a port within an AI system component, including at least one of a union, tee, cross, pump, valve, column, guard column, manifold, or detector.

In some embodiments, a fluidic connection assembly is provided which comprises a first body having a passageway therethrough and having first end and a second end, and having a plurality of tabs defining the second end, wherein a middle portion of said first body has a plurality of radially inwardly extending shoulder portions, each corresponding to one of the tabs, wherein each of the shoulder portions are adapted to move radially inwardly when a corresponding tab is moved radially inwardly, a second body having a first end and a second end and located within said first body, wherein the first end of said second body abuts the interior surface of the first end of said first body, said second body further having a plurality of middle portions, each adapted to move radially inwardly when a corresponding shoulder portion of said first body moves radially inwardly, and further having latch portions proximal the second end of said second body, wherein the latch portions are adapted to move radially inwardly when the middle portions move radially inwardly, and wherein the latch portions are adapted to removably engage with radially inward projections at a first end of a port body in an AI system, said second body further having a passageway therethrough adapted to receive a tube extending through said second body, and a spring located in said second body and having a first end proximal the first end of said second body and a second end adapted to exert a force to urge one end of the tube against at least a portion of a port in the port body. The assembly may further comprise a spring and a tube extending through said spring, and the end of said tube may comprise a PEEK tip. In addition, the assembly may further comprise a boss in the port of the port body which has a diameter smaller than the outer diameter of the tube. The port may comprise a port within an AI system component, including at least one of a union, tee, cross, pump, valve, column, guard column, manifold, or detector.

In certain embodiments, a fluidic connection assembly is provided which may comprise a body having a first end and a second end, each of the first and second ends having openings therein, and having a hollow portion therein, a first spring located at least partially within the hollow portion of said body, and having a first end abutting an interior surface of the hollow portion of said body, a latch member having an opening therethrough and having a base portion and a top portion, wherein the base portion of said latch member is adapted to fit at least partially within said body, and wherein the top portion has one or more projections adapted to be held in recesses on an exterior portion of said body, and a cap member having an opening therethrough and having first and second sides and an extension on the first side of said cap member which is adapted to extend into at least a portion of the hollow portion of said body and to hold said latch member and said cap member together, wherein the second side of said cap member is adapted to be attached to the second end of said body, and wherein said latch member is adapted to engage with and securely hold an adapter when a portion of one end of the adapter is inserted into the opening of said cap member and the opening of said latch member, and wherein each of said body, said spring, said latch member, and said cap member are adapted to receive at least a portion of tubing therethrough. In addition, the first spring may be in a compressed state when said cap member, said latch member, and said body are assembled together and said first spring is located entirely within the hollow portion of said body. The fluidic connection assembly may further comprise a second spring, wherein said second spring is located within the hollow portion of said body and adjacent to at least one side of said latch member. The second spring may be adapted to push said latch member so that a radially inward projection of said latch member is pressed against a portion of said adapter. The adapter and the inward projection of the latch member may have cooperating ramps or angled portions to allow for easier engagement of the adapter and the inward projection. The fluidic connection assembly may further comprise a tube having one end which extends through the first and second ends of said body, said first spring, said latch member, and extends out of the opening of the second side of said cap member. The first spring may exert a force which urges the end of said tube away from said body. The fluidic connection assembly may further comprise a washer or disc located within said body and adjacent to a second end of said first spring, a sleeve member surrounding at least a portion of said tube, wherein said sleeve member has a first end and a second end, and wherein said first spring exerts a force on said washer and said washer exerts a force on said sleeve, and said sleeve exerts a force on said tube which urges the end of said tube away from said body. The assembly may further comprise a tip surrounding a portion of the tube and at least a portion of which is surrounded by the sleeve, wherein the force exerted on the sleeve is transferred to one end of said tip to urge said tip against a surface of a port. The port may comprise a port within an AI system component, including at least one of a union, tee, cross, pump, valve, column, guard column, manifold, or detector.

In certain embodiments, a fluidic connection assembly is provided which may comprise a body having a first end and a second end, each of the first and second ends having openings therein, and having a hollow portion therein, a first spring located at least partially within the hollow portion of said body, and having a first end abutting an interior surface of the hollow portion of said body, a latch member having an opening therethrough and having a base portion, and a cap member having an opening therethrough and having first and second sides, and which is adapted on one side to surround a portion of one end of the body, with the cap further having a base portion which is adapted to receive and hold a second spring therein, with the cap further adapted to receive and hold at least a portion of the latch member therein, with the latch member further adapted to engage with and securely hold an adapter when a portion of one end of the adapter is inserted into the opening of said latch member, and wherein each of said body, said spring, said latch member, and said cap member are adapted to receive at least a portion of tubing therethrough. In addition, the first spring may be in a compressed state when said cap member, said latch member, and said body are assembled together and said first spring is located entirely within the hollow portion of said body. The fluidic connection assembly may further comprise a second spring, wherein said second spring is located within the base portion of said cap and adjacent to at least one side of said latch member. The second spring may be adapted to push said latch member so that a radially inward projection of said latch member is pressed against a portion of said adapter and/or into an annular notch of the adapter. The adapter and the inward projection of the latch member may have cooperating ramps or angled portions to allow for easier engagement of the adapter and the inward projection. The fluidic connection assembly may further comprise a tube having one end which extends through the first and second ends of said body, said first spring, said latch member, and extends out of the opening of the second side of said cap member. The first spring may exert a force which urges the end of said tube away from said body. The fluidic connection assembly may further comprise a washer or disc located within said body and adjacent to a second end of said first spring, a sleeve member surrounding at least a portion of said tube, wherein said sleeve member has a first end and a second end, and wherein said first spring exerts a force on said washer and said washer exerts a force on said sleeve, and said sleeve exerts a force on said tube which urges the end of said tube away from said body. The assembly may further comprise a tip surrounding a portion of the tube and at least a portion of which is surrounded by the sleeve, wherein the force exerted on the sleeve is transferred to one end of said tip to urge said tip against a surface of a port. The port may comprise a port within an AI system component, including at least one of a union, tee, cross, pump, valve, column, guard column, manifold, or detector.

In certain embodiments a method of connecting a tube to a port is provided, with the method comprising the steps of providing a tube having first and second ends, inserting the first end of the tube through a fluidic connection assembly which may comprise a body having a first end and a second end, each of the first and second ends having openings therein, and having a hollow portion therein, a first spring located at least partially within the hollow portion of said body, and having a first end abutting an interior surface of the hollow portion of said body, a latch member having an opening therethrough and having a base portion and a top portion, wherein the base portion of said latch member is adapted to fit at least partially within said body, and wherein the top portion has one or more projections adapted to be held in recesses on an exterior portion of said body, and a cap member having an opening therethrough and having first and second sides and an extension on the first side of said cap member which is adapted to extend into at least a portion of the hollow portion of said body and to hold said latch member and said cap member together, wherein the second side of said cap member is adapted to be attached to the second end of said body, and wherein said latch member is adapted to engage with and securely hold an adapter when a portion of one end of the adapter is inserted into the opening of said cap member and the opening of said latch member, and wherein each of said body, said spring, said latch member, and said cap member are adapted to receive at least a portion of tubing therethrough, inserting a least a portion of tube into an adapter and inserting at least a portion of one end of the adapter into the second end of said body and having the projection of said latch member engage with a portion of the adapter to securely hold the adapter and the latch member together, and engaging the other end of the adapter with a port, such as by threadably engaging threads on the adapter with threads of the port.

### BRIEF DESCRIPTION OF THE DRAWINGS

FIG. 1 is an isometric view of a fluidic connector assembly in accordance with an embodiment of the present disclosure.
FIG. 2 is a front view of a fluidic connector assembly in accordance with an embodiment of the present disclosure.
FIG. 3 is a cross-sectional view of the fluidic connector assembly shown in FIG. 2 taken along line C--C.
FIG. 4A is an enlarged cross-sectional view of a portion of the fluidic connector assembly shown in FIGs. 2 and 3.
FIG. 4B is an isometric view of a band spring useful in an embodiment of the present disclosure.
FIG. 5 is a side view of a fluidic connector assembly in accordance with an embodiment of the present disclosure.
FIG. 6 is a cross-sectional view of the fluidic connector assembly shown in FIG. 5 taken along line D--.
FIG. 7 is a cross-sectional view of an embodiment of a fluidic connector assembly which is connected to a port.
FIG. 8 is an enlarged cross-sectional view of a portion of the fluidic connector assembly shown in FIG. 8.
FIG. 9 is a cut-away view of an embodiment of a fluidic connector assembly in accordance with the present disclosure.
FIG. 10 is a cross-sectional view of the fluidic connector assembly shown in FIG. 9.
FIG. 11 is a cross-sectional view of a fluidic connector assembly in accordance with an embodiment of the present disclosure, and is shown in a connected state.
FIG. 12 is an enlarged, cross-sectional view of a portion of the fluidic connector assembly shown in FIG. 11
FIG. 13 is an enlarged, cross-sectional view of a portion of a fluidic connector assembly in accordance with an embodiment of the present disclosure.
FIG. 14 is an enlarged, cross-sectional view of a portion of a fluidic connector assembly in accordance with an embodiment of the present disclosure.
FIG. 15 is an enlarged, cross-sectional view of a portion of a fluidic connector assembly in accordance with an embodiment of the present disclosure.
FIG. 16 is an enlarged, cross-sectional view of a portion of a fluidic connector assembly in accordance with an embodiment of the present disclosure.
FIG. 17 is a cross-sectional view of a fluidic connector assembly in accordance with one embodiment of the present disclosure, and is shown in a connected state.
FIG. 18 is an enlarged cross-sectional view of a portion of a fluidic connector assembly in accordance with one embodiment of the present disclosure, and is shown in a connected state.
FIG. 19 is another enlarged cross-sectional view of a portion of the fluidic connector assembly shown in FIG. 18 in accordance with one embodiment of the present disclosure, and is shown in a connected state.
FIG. 20 is another enlarged cross-sectional view of a portion of the fluidic connector assembly shown in FIG. 17 in accordance with one embodiment of the present disclosure, and is shown in a connected state.
FIG. 21 is an isometric view of another alternative embodiment of an assembly in accordance with the present disclosure.
FIG. 22 is a side view of the assembly of FIG. 21.
FIG. 23 is a cross-sectional view of the assembly of FIG. 21 taken along line E-E of FIG. 22.
FIG. 24 is another side view of the assembly of FIG. 21.
FIG. 25 is a cross-sectional view of the assembly of FIG. 21 taking along line F-F of FIG. 24.
Fig. 26 is a cross-sectional view of a connection assembly in accordance with an embodiment in accordance with the present disclosure.
Fig. 27 is a cross-sectional view of a connection assembly and a port configuration in accordance with an embodiment in accordance with the present disclosure.
Fig. 28 is a cross-sectional view of a connection assembly and a port configuration in accordance with an embodiment in accordance with the present disclosure.
Fig. 29 is a cross-sectional view of a connection assembly and a port configuration in accordance with an embodiment in accordance with the present disclosure.
Fig. 30 is a cross-sectional view of a connection assembly in accordance with an embodiment in accordance with the present disclosure.
Fig. 31 is a cross-sectional view of a connection assembly and a port configuration in accordance with an embodiment in accordance with the present disclosure.
Fig. 32 is an exploded view of a connection assembly of an alternative embodiment in accordance with the present disclosure.
Fig. 33 is a partial cross-sectional view of the connection assembly of Fig. 32 in a connected configuration in accordance with the present disclosure.
Fig. 34 is an exploded view of a connection assembly of an alternative embodiment in accordance with the present disclosure.
Fig. 35 is a partial cross-sectional view of the connection assembly of Fig. 33 in a connected configuration in accordance with the present disclosure.
Fig. 36 is a partial cross-sectional view of a tubing assembly in accordance with one embodiment of the present disclosure.
Fig. 37 is an enlarged partial cross-sectional view of the tubing assembly of Fig. 36.
Fig. 38A is a frontal view of a tip of the tubing assembly of Figs. 36 and 37.
Fig. 38B is a side view of a tip of the tubing assembly of Figs. 36 and 37.
Fig. 38C is a cross-sectional view of the tip of the tubing assembly of Figs. 36 and 37.
Fig. 39 is a cross-sectional view of a quick connect/disconnect assembly in a connection assembly in one embodiment of the present disclosure.
Fig. 40 is an exploded view of the assembly of Fig. 39.
Fig. 41 is a view of the assembly of Figs. 39 and 40 shown in an exploded cross-sectional view with an adapter and a union in an embodiment of the present disclosure.
Fig. 42 is an exploded view of a fluidic connection assembly of one particular embodiment in accordance with the present disclosure and particularly in accordance with the invention.
Fig. 43 is a partially exploded three-dimensional view of a fluidic connection assembly of one particular embodiment in accordance with the present disclosure and particularly in accordance with the invention.
Fig. 44 is a three-dimensional view of the fluidic connection assembly of Fig. 43 in an assembled state.
Fig. 45 is a cross-sectional view of the fluidic connection assembly of Fig. 43.
Fig. 46 is a cross-sectional view of the fluidic connection assembly shown in Fig. 45 as shown in an assembled state.

### DETAILED DESCRIPTION

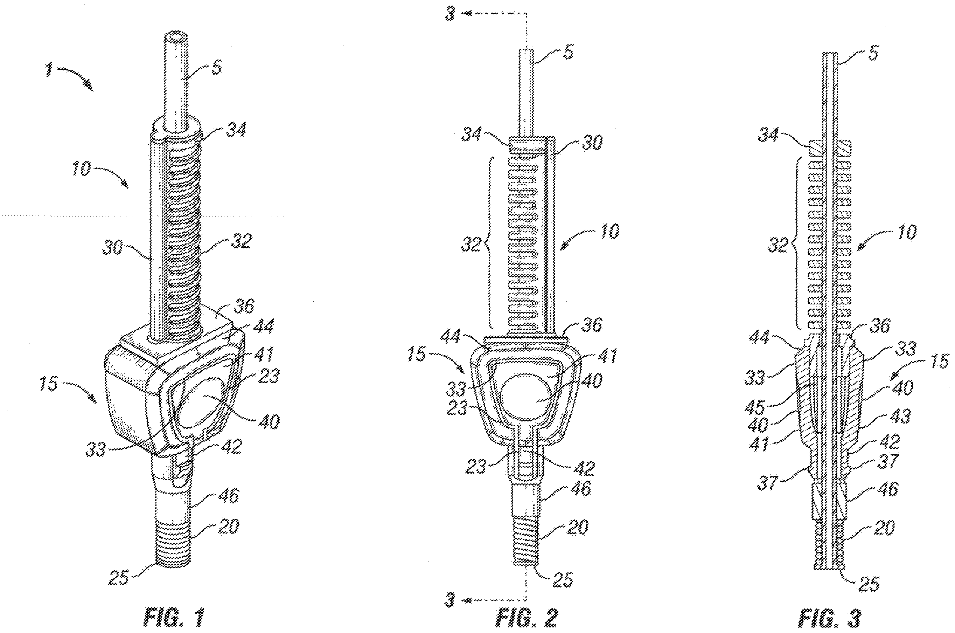

FIG. 1 provides an isometric view of a fluidic connector assembly 1 in accordance with one embodiment of the present disclosure. As shown in FIG. 1, the fluidic connector 1 and its components are assembled. As shown in FIG. 1, the assembled fluidic connector assembly **1** includes tubing **5,** a top extension **10,** a body **15,** a spring **20,** and a bottom flange **25.** In this particular embodiment, the tubing **5** extends through passageways adapted to receive the tubing **5** in each of the extension **10** and body **15,** and the tubing **5** extends through the center open area of the spring **20,** which in this particular embodiment is a coiled spring. In this particular embodiment, the bottom flange **25** of the fluidic connector assembly **1** is provided by one end of tubing **5.** Each of these main components of the fluidic connector **1** and their use and operation are described in more detail below.

Still referring to FIG. 1, the extension portion **10** includes a member **30** which extends longitudinally on one side of the extension **10** from a top end to a base member **36.** Extension member **10** also includes a series of toroidal, or ring-like, members **32.** As shown in FIG. 1, the toroidal members **32** generally form rings around, and are generally disposed perpendicular to, the longitudinal axis of the extension **10.** At the top end of the extension member **10** is a top toroidal member **34,** which is larger in size than the other toroidal members **32** of the extension **10.** Those skilled in the art will appreciate that the toroidal members **32** may vary in size, shape, and number as may be desired. In addition, the toroidal members **32** could be replaced by a solid column (not shown) if desired, or by a column with slots, grooves, holes, or the like therein as may be desired. The extension member **10** provides an advantage because it provides relief to tubing **5** to avoid unnecessary strains or stresses imposed on tubing **5** if twisted, grabbed, or otherwise manipulated by an operator. The top extension **10** thus protects the tubing 5 to avoid wear and tear. To a lesser extent, an operator may grasp and manipulate the extension **10.** Those skilled in the art will appreciate that the extension **10** can be provided in other forms as may be desired to provide relief for tubing **5,** and will also appreciate that the assembly **1** can be provided either with or without an extension **10,** whether in the specific embodiment shown and disclosed herein or another form or shape. As also shown in FIG. 1, the base **36** of the extension **10** is located on top of the body **15.** The body **15** includes a top portion **44,** a face **41,** a hinge **33,** and an indentation **40** on face **41,** as well as a bottom member **42.** Details regarding the body **15** and its features and operation are provided below.

As shown in FIG. 1, located below the bottom member **42** of body **15** is a ring portion **46** of the body **15** and, located below the ring portion **46,** is a spring member **20.** The spring member **20** in this particular embodiment can be a coiled spring of a desired material, length, and strength. Those skilled in the art will appreciate that the material and size of spring member **20** can be selected so that the spring member **20** provides the desired amount of force, or biasing, when engaged (as described in more detail below) in a fluidic connection in a desired application. For example, one of skill would appreciate that it would be advantageous to have the spring member **20** provide a greater biasing force when the fluidic connector **1** will be used in an application with a greater fluidic pressure, than might be the case if the fluidic connector **1** is to be used in an application with a relatively small fluidic pressure. For example, not all fluidic connections in a given AI system used for a given application need operate under the same fluid pressure. The fluid pressures may change even in such situations for different connections in the AI system. Moreover, a given AI system may be used in an application in which lower fluid pressures are used than may be encountered in the same AI system when used for a different application. For example, an LC system may be used for in vitro applications with fluid pressures at 200 psi or less, yet this same system may also be used in other applications with higher pressures.

Also shown in FIG. 1 is a flange **25** of tubing **5.** As can be seen, the flange **25** is located at the bottom of the fluidic connector **1,** and below the bottom end of the spring **20.** In the particular embodiment shown in FIG. 1, the flange **25** is one end of the tubing **5,** and tubing **5** extends from flange **25** through the spring **20,** body **15,** and extension **10.** The flange **25** provides an advantage because it is located between a port or other connector (not shown in FIG. 1) when the fluidic connector **1** is connected to a port or other connector and the spring **20.** This is useful, for example, in systems involving biological samples, because the flange **25** and tubing **5** can be made of a biocompatible material and spring **20** can be made of metal if desired, as the spring **20** is not located in the flow path and thus cannot contaminate or otherwise affect the fluid passing through the system in which the fluidic connector **1** is to be used.

Referring now to FIGs. 2 and 3, additional details regarding the fluidic connector **1** and its components and features are shown. For ease of reference, the same numbers are used in the various Figures for the same items. FIG. 2 is a "front" view of the fluidic connector **1,** while FIG. 3 is a cross-sectional view of the fluidic connector **1** taken along line C-C of FIG. 2. Those skilled in the art will appreciate that terms such as "top," "bottom," "front," "back," and the like as used herein are for convenience in reference to the Figures, but in fact the fluidic connector **1** can be used in any orientation, so such terms should not be considered limiting in any fashion but are instead merely for the convenience of the reader.

As shown in FIGs. 2 and 3, the tubing **5** extends along a longitudinal axis of the fluidic connector **1** as assembled. Each of the extension **10,** body **15,** and spring **20** are configured so that each has a passageway through which the tubing **5** may extend. As shown in FIG. 3, the body **15** has a first side **41** and a second side **43,** and in this particular embodiment, each has an indentation **40.** Those skilled in the art will appreciate that, although both sides **41** and **43** of the body **15** are substantially the same as one another, this need not be the case, such as if a particular application in which the fluidic connector **1** is to be used presents a configuration such that a different shape or size of body **15** is more appropriate. As also shown in FIG. 3, a portion of the base **36** of the extension **10** extends into an interior portion of the body **15** at the top end of the body **15.** Those skilled in the art will appreciate that the extension base **36** and the body **15** can be adapted to allow the extension base **36** to fit securely and/or releaseably in the top end of the body **15.** Alternatively, a portion of the extension base **36** can be secured to the interior of the body **15,** such as by use of a glue or adhesive, or the outer diameter of the portion of the base **36** to fit inside the interior portion of the body **15** can be substantially the same size or slightly larger if the material of the extension base **36** can be deformed as the inner diameter of the body **15** in the area adapted to securely receive that portion of the extension base **36.** Those skilled in the art will appreciate that, because the extension **10** need not be used if so desired, the base **36** need not form a portion of extension **10** and, indeed, the connection assembly **1** and the body **15** do not require the use of the base **36.** For example, the top of the body **15** in place of a base **36** could instead be formed of the same material as the rest of body **15,** or could be left open if desired. Those of skill will also appreciate that the body **15** and base **36** can be formed to allow a snap-fit connection, such that the base **36** and extension **10** can be removably attached to the body **15** by an operator, or detached therefrom, as may be desired.

A hinge **33** is located at or near the top of the face **41** and at or near the top of face **43.** The hinges **33** allow at least portions of the faces **41** and **43** to move inwardly and outwardly with respect to the longitudinal axis of the fluidic connector **1.** Referring to FIG. 2, the hinge **33** at or near the top of the body **15** can be seen. In this particular embodiment, the hinge **33** defines the top of the face **41.** In addition, a slot **23** on each side of the face **41** extends from each side of the hinge **33** downward and defines the face **41.** As shown in FIG. 2, the slots **23** further extend downwardly from face **41** on either side of a front face of the bottom member **42** of the body **15.** As can be seen from FIGs. 2 and 3, when the face **41** is pushed inwardly by an operator towards the longitudinal axis of the assembly **1,** the portion of face **41** below hinge **33** can move inward because it is not fixed with respect to the rest of body **15.** In addition, it can be seen that the two slots **23** join together near the bottom of the bottom member **42** of the body **15.** The slots **23** are located and define the area of face **41** that can move when pressed such that the projections or latches **37** also move inwardly with the face **41.** It will be appreciated, such as from FIG. 3, for example, that when the latches **37** both move inwardly, the distance between their outer edges decreases and provides a shorter length, thus allowing easy and quick insertion of the bottom member **42** of body **15** into, or removal from, a port of an AI system component. Those skilled in the art will appreciate that, although slots **23** as shown in FIG. 2, for example, extend continuously from either side of hinge **33** and define face **41** and then meet and join at or near the bottom of the bottom member **42,** slots **23** can be configured differently if desired. For example, face **41** can be defined by a series of two or more slots (not shown) that are not continuous but yet extend in essentially the same pattern as shown in FIG. 2 for slots **23.** Alternatively, instead of slots **23,** a weakened area of the body **15** may be provided, such as the case described above for hinge **33** with a thinner width, to define face **41.**

As shown in FIG. 3 (and as described in more detail below), a second spring member **45** is located within body **15** in this embodiment. In a normal resting position (*e.g.*, without any force applied to faces **41** and **43** by an operator), the spring member **45** exerts a force against the inside surfaces of faces **41** and **43** and pushes them away from the longitudinal axis and to a closed position, such as is shown in FIG. 2. When a force is exerted on the faces **41** and **43,** pushing them towards the longitudinal axis of the fluidic connector **1,** the projections **37** proximal the bottom of the body **15** move towards the longitudinal axis, thereby making it easier for an operator to quickly insert the projections **37** (and thereby a portion of the fluidic connector **1**) into a port or fitting (not shown in FIGs. 2 and 3), or to remove the projections **37** (and thereby the fluidic connector **1**) from a port or fitting.

Referring now to FIGs. 4A and 4B, additional details regarding the fluidic connector assembly **1** shown in FIGs. 2 and 3, and particularly with respect to body **15,** are shown and are described below. FIG. 4A provides an enlarged, cross-sectional view of a portion of the fluidic connector **1,** primarily the body **15.** As shown in FIG. 4A, the tubing **5** extends through passageways extending through the extension base **36** and the body **15.** As in FIG. 4, the body **15** in FIG. 4A includes a left indentation **40** on the left face **41** and a right indentation **40** on the right face **43** of body **15.** As in FIG. 4, a portion of the extension base **36** is located within an interior portion of the body **15,** at the top end of body **15,** and a portion of the extension base **36** extends outward and exterior to the top end of the body **15.** Located below the portion of the extension base **36** which is located within an interior portion of body **15** is a second spring member **45.**

As shown in FIG. 4A, the tubing **5** also extends through the spring member **45** and along the longitudinal axis thereof. FIG. 4B provides an isometric view of the second spring member **45.** As shown in FIG. 4B, the second spring member **45** in this particular embodiment is a band spring with a slot running the vertical length thereof. The second spring member **45** serves to bias or push the two faces **41** and **43** outwardly and away from the longitudinal axis of the assembly **1,** but is selected and adapted (such as by the selection of its shape, material(s) and size) so that it can be deformed (from the position shown in FIGs. 4A and 4B) inwardly when a force is applied, such as when a user presses the two indentations **40** on the two faces **41** and **43** towards the longitudinal axis of the fluidic connector **1.** Those skilled in the art will appreciate that the assembly **1** can be usefully used without the need for a spring **45.** However, the use of a spring **45** is believed to be useful in order to bias the faces **41, 43** outwardly and to thereby avoid potential problems with fatigue of the material of the body **15.** For example, if no spring **45** is used and the body **15** and faces **41, 43** have been pushed inwardly numerous times, the faces **41, 43** may tend to stay inwardly disposed, which may not be desired. The use of spring **45** thus can be used to help provide biasing of faces **41, 43** outwardly as may be desired.

Those skilled in the art will appreciate that human strength may vary, sometimes substantially. A fluidic connector assembly which is easily and quickly operable by men and women, strong or weak, would be advantageous. Such an assembly also would be advantageous if the ability to obtain a sealed connection does not vary from user to user, and does not require a narrow range of force or torque to obtain a sealed engagement, and does not require the use of any tools. The fluidic connector assembly **1** provides these advantages. For example, spring member **45** can be selected to require no more than an amount in a range of from two to five (e.g., three) pounds of force to be applied to indentations **40** to move the projections **37** inwardly enough to detach the fluidic connector **1** from a port. Almost all reasonably healthy and non-disabled adult humans are believed able to exert such a force by pinching sides **41** and **43** together between a thumb and finger (and without requiring any tools). Moreover, the fluidic connector assembly **1** of the present disclosure is advantageous because, once connected to a port, the fluidic connector assembly **1** provides a secure sealing engagement. An operator can easily visually check to be sure that the projections **37** are held in a port; such a confirmation is all that is needed for the operator to confirm that a seal has been obtained with the connection. Moreover, the sides **41** and **43** will provide a visual cue and confirmation of a sealing engagement because, once the sealing engagement has been made, the latches **37** will be biased outwardly from the longitudinal axis, and so will the faces **41** and **43,** with the location of the faces **41** and **43** easily visible to an operator.

Referring now to FIGs. 5 and 6, additional views of the fluidic connector assembly **1** in connection with the embodiment described above are provided. FIG. 5 provides a side view of the fluidic connector **1,** as well as a cutline D - D, and FIG. 6 provides a cross-sectional view of the fluidic connector **1** as taken along line D - D as indicated in FIG. 5. It will be appreciated that additional details regarding the shapes and features of the fluidic connector **1** can be seen. For example, FIG. 6 provides a cross-sectional view showing spine member **30** of the extension **10** on one side of the extension **10** with the series of rings or toroidal members **32** on the other side.

Those skilled in the art will appreciate that, depending on the intended application, including without limitation the expected fluidic pressures, the nature of the fluids to be used, the nature of the samples to be analyzed, and the like, the composition of the materials used, as well as the specific shapes and sizes, of the various components and features of the fluidic connector **1** as shown and described above can be varied. For example, in applications in which biocompatibility is desired, the tubing **5** can be made of polyetheretherketone (PEEK), polyaryletherketone (PAEK), polyetherketoneketone (PEKK), fluorinated ethylene propylene (FEP), ethylene tetrafluoroethylene (ETFE), polytetrafluoroethylene (PTFE), perfluoroalkoxy (PFA, also called perfluoroalkoxyethylene), polychlorotrifluoroethylene (PCTFE), polymer-sheathed fused silica (such as PEEKSil), fused silica, or silica borite, and can further include a filler material, which can include fibers, such as carbon fibers, glass fibers, nanofibers, and/or metallic fiber, depending on the pressures and fluids involved. It is anticipated that the fluidic connector 1 will be of particular usefulness in in vitro applications, such as those in which the fluidic pressures are about 200 psi or less, and in which biological materials are involved. In such situations, it is often desirable for the fluid path to contain only biocompatible materials. In the embodiment shown and described, however, this is achieved because the tubing **5** is the only component of the fluidic connector **1** touching the fluid. In the embodiment shown in FIGs. 1-6, then, the spring **20** and the second spring member **45** can be made of metal, such as stainless steel, titanium, steel, nickel, nitinol. Alternatively, the spring **20** and/or the second spring member **45** can be made of PEEK, PEI, and/or PPSU.

The extension **10** and body **15** can be made of any one of a number of different materials, including any one or more of the following: metal, such as steel, including stainless steel, aluminum, titanium, as well as polymeric materials such as polyetheretherketone (PEEK), polyoxymethylene (POM) such as available under the mark DELRIN and known sometimes as acetal, RADEL brand polyphenylsulfone (PPSU), ULTEM brand polyetherimide (PEI), polyetherketoneketone (PEKK), polyaryletherketone (PAEK), polyethylene, polypropylene, polyvinylchloride, acrylic, and/or other materials, such a fused silica, silica borite, PEEKsil, and the like. In one embodiment, the extension is made of one or more polymeric materials such as polyetheretherketone (PEEK), polyoxymethylene (POM) such as available under the mark DELRIN and known sometimes as acetal, RADEL brand polyphenylsulfone (PPSU), ULTEM brand polyetherimide (PEI), polyetherketoneketone (PEKK), polyaryletherketone (PAEK), polyethylene, polypropylene, polyvinylchloride, acrylic, and/or other polymeric materials, with a wire made of a metal located within the spine member **30** and over molded with PEEK, although those skilled in the art will appreciate that any one of a number of additional or different polymers may be used for this purpose. This particular embodiment of the spine member **30** of the extension **10** allows an operator to bend or twist or otherwise shape the extension **10** or a portion thereof, and for that portion of the extension so manipulated by an operator to retain its shape indefinitely.

As noted above, the extension **10** can be optional. If included, extension **10** can be integral with the body **15,** or the extension **10** can be a separate piece that is attached to the body **15.** The extension **10** can be permanently attached to the body, such as by glue, epoxy, or other adhesive means, or can be attached such as by melting either or both of extension **10** and body **15** and attaching them and then allowing the polymeric material(s) to harden, or by sintering or other methods. In addition, the extension **10** and body **15** can be adapted so that the extension **10** can be removably attached to the body **15,** such as by providing latching means or a compression fit. For example, the extension **10** can be adapted so that its base end has a slightly larger area than the top of the body **15** and can be compressed by a user, then inserted into the top of the body **15,** such that when the operator releases the pressure on the extension **10** base end, it expands and is removably secured to the body **15.**

An embodiment of the operation of the fluidic connector **1** is now described with reference to FIGs. 2 and 3, although those skilled in the art will appreciate the operation of the fluidic connector **1** in connection with all of the FIGs. An operator can assemble the fluidic connector **1** by placing the tubing **5** through the center passageways of the spring **20,** the body **15,** and the extension **10.** (As noted above, the extension **10** and body **15** can be securely joined together or can be removably joined.) It will be appreciated that the spring **20,** when compressed, will exert a force against the flanged end **25** of the tubing **5** and also against the bottom ring **46** of the body **15.** This is described in more detail below in connection with the use of the fluidic connector **1** in a port.

As shown in FIG. 3, indentations **40** are located on opposing faces **41** and **43** of the body **15.** Although shown as circular, they need not be. It will be appreciated that the spring **45** (shown in more detail in FIGs. 4A and 4B) can be used to provide a force biasing the faces **41** and **43** towards an outer or exterior position (*i.e.,* away from the longitudinal axis of fluidic connection **1**). The faces **41** and **43** can be depressed by an operator (without the use of tools), thus forcing the spring **45** to compress towards the longitudinal axis of the fluidic connector **1.** As the operator presses the faces **41** and **43** towards the longitudinal axis of the fluidic connector **1,** the spring **45** compresses on opposing sides as the faces **41** and **43** push the spring **45** inwardly towards the longitudinal axis of the fluidic connector **1.** As the spring **45** is compressed inwardly, the bottom member **42** of the body **15** moves inwardly from its previously biased position. When this happens, the latches or projections **37** on the bottom member **42** also move inwardly and thus have a smaller outer diameter or length, thereby allowing quick insertion into, and easy removal of the fluidic connector **1** from, a port or other fluidic connection, such as in a component in an AI system.

By pressing faces **41** and **43** inwardly and inserting the bottom portion of assembly **1** into a port, then releasing the pressure on faces **41** and **43,** an operator can obtain a connection that is sealed and leak-proof, and provides sufficient force to keep the sealed connection (even when a fluid under high pressures, such as from about 200 psi to about 5000 psi or so is flowing through the tubing **5**) in place. An operator can make a desired connection in a LC or other analytical instrument system using the assembly **1** without the need for any torque, such as on a nut, without the need for a threaded connection, and without the need for any additional tools, such as a wrench or the like. Moreover, an operator can disconnect the assembly **1** from a port by pressing faces **41** and **43** inwardly and pulling on the body **15** or other portion of the assembly **1** and easily removing the assembly **1** from the port, again without the need for any application of a torque, without the need for disconnecting a threaded engagement (such as if a nut with threads is used), and without the need for any additional tools.

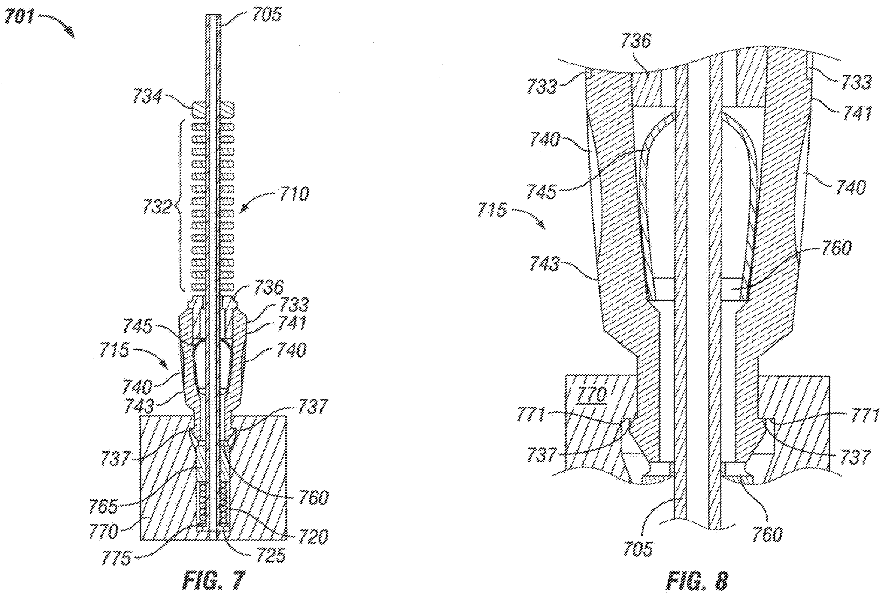

Referring now to FIGs. 7 and 8, an alternative embodiment of a fluidic connector **701** is shown. In FIG. 7, the fluidic connector **701** includes tubing **705** which extends along and around a longitudinal axis of the fluidic connector **701.** The fluidic connector **701** includes an extension member **710,** a body **715,** a first ring **760,** a second ring **765,** a spring **720,** and a backup ring **775.** The extension member **710** can be substantially the same as the extension member **10** described above, and as shown in FIG. 7, the extension member **710** includes a series of toroidal members **732** located between an extension base **736,** and a top ring or toroidal member **734.**

The body **715,** as shown in FIG. 7, includes hinges **733** on opposing faces **741** and **743.** Each of the faces **741** and **743** includes an indentation **740.** Located within an interior portion of the body **715** is a spring member **745.** As shown in FIG. 7, the spring member **745** in this particular embodiment is a cantilevered spring, which can be made of metal or other materials, such as those described above. It can be seen from FIG. 7 that the spring **745** can be selected with respect to its size, shape and material composition so that the spring **745** exerts a force outward from the longitudinal axis of the fluidic connector **701** and against the faces **741** and **743.**

Still referring to FIG. 7, it can be seen that the lower portion of the body **715** is located within a port in a body **770.** The port in the body **770** can be a port in any one of a number of components or fittings in an AI system. As shown in FIG. 7, the body **715** has projections **737** which have a greater outer diameter than the portions of the body **715** above and below the projections **737.** The projections **737** hold the lower portion of the body **715** within the port, since the port opening has a diameter less than that of the outer diameter of the projections **737** when the body **715** is in a normal or rest position.

The fluidic connector **701** shown in FIG. 7 also includes a first ring **760,** a second rig **765,** and a spring **720.** The spring **720** in this particular embodiment and as shown in FIG. 7 is a coiled spring, which can be made of metal or other appropriate materials, such as those discussed above. The spring **720** can be selected as to its size, shape, and material composition so that it exerts a desired force against both upwards and downwards. Because the projections **737** hold the body **715** in place once they are located as shown in FIG. 7 with respect to the port of body **770,** the body **715** is securely connected to the body **770.** In addition, the spring **720** exerts a downward force, thereby pressing downwards on the backup ring **775** which, in turn, presses downwardly on the flange **725** of the tubing **705,** thus keeping the end of the flange **725** of the tubing **705** pressed firmly and sealingly against the passageway in the body **770.**

Referring now to FIG. 8, an enlarged cross-sectional view of a portion of the body **715** and certain components is provided. As shown at the top end of the body **715,** a portion of the base **736** of the extension member **710** is located within and proximal to a top end of the body **715.** Hinges **733** are located in the body **715,** also proximal to its top end, but as shown in FIG. 8, the hinges **733** are located on opposing faces **741** and **743** of the body **715.**

Referring now to FIGs. 7 and 8, an alternative embodiment of a fluidic connector **701** is shown. In FIG. 7, the fluidic connector **701** includes tubing **705** which extends along and around a longitudinal axis of the fluidic connector **701.** The fluidic connector **701** includes an extension member **710,** a body **715,** a first ring **760,** a second ring **765,** a spring **720,** and a backup ring **775.** The extension member **710** can be substantially the same as the extension member **10** described above, and as shown in FIG. 7, the extension member **710** includes a series of toroidal members **732** located between an extension base **736,** and a top ring or toroidal member **734.**

The body **715,** as shown in FIG. 7, includes hinges **733** on opposing faces **741** and **743.** Each of the faces **741** and **743** includes an indentation **740.** Located within an interior portion of the body **715** is a spring member **745.** As shown in FIG. 7, the spring member **745** in this particular embodiment is a cantilevered spring, which can be made of metal or other materials, such as those described above. It can be seen from FIG. 7 that the spring **745** can be selected with respect to its size, shape and material composition so that the spring **745** exerts a force outward from the longitudinal axis of the fluidic connector **701** and against the faces **741** and **743.**

Still referring to FIG. 7, it can be seen that the lower portion of the body **715** is located within a port in a body **770.** The port in the body **770** can be a port in any one of a number of components or fittings in an AI system. As shown in FIG. 7, the body **715** has projections **737** which have a greater outer diameter than the portions of the body **715** above and below the projections **737.** The projections **737** hold the lower portion of the body **715** within the port, since the port opening has a diameter less than that of the outer diameter of the projections **737** when the body **715** is in a normal or rest position.

The fluidic connector **701** shown in FIG. 7 also includes a first ring **760,** a second rig **765,** and a spring **720.** The spring **720** in this particular embodiment and as shown in FIG. 7 is a coiled spring, which can be made of metal or other appropriate materials, such as those discussed above. The spring **720** can be selected as to its size, shape, and material composition so that it exerts a desired force against both upwards and downwards. Because the projections **737** hold the body **715** in place once they are located as shown in FIG. 7 with respect to the port of body **770,** the body **715** is securely connected to the body **770.** In addition, the spring **720** exerts a downward force, thereby pressing downwards on the backup ring **775** which, in turn, presses downwardly on the flange **725** of the tubing **705,** thus keeping the end of the flange **725** of the tubing **705** pressed firmly and sealingly against the passageway in the body **770.**

Referring now to FIG. 8, an enlarged cross-sectional view of a portion of the body **715** and certain components is provided, with the body **715** connected to a port of a body **770.** As shown at the top end of the body **715,** a portion of the base **736** of the extension member **710** is located within and proximal to a top end of the body **715.** Hinges **733** are located in the body **715,** also proximal to its top end, but as shown in FIG. 8, the hinges **733** are located on opposing faces **741** and **743** of the body **715.** Hinges **733** are adapted to allow the faces **741** and **743** to move inwards towards the longitudinal axis of the fluidic connector **701** when a force is exerted thereon, such as when the faces **741** and **743** are pressed by an operator. When such a force is exerted, such as when an operator presses on the faces **741** and **743,** and the faces move inwardly, the projections **737** also move inwardly towards the longitudinal axis. Thus, an operator can remove the fluidic connector **701** from the body **770,** and disconnect the fluidic connector **701,** by pressing on the faces **741** and **743** and pulling the fluidic connector **701** upwards and away from the port of the body **770.** Because the tubing **705** extends through passageways in each of the other components of the fluidic connector **701** as shown in FIG. 7, the entire fluidic connector **701** can be removed from the body **770** without any need to disassembly or remove any of the components of the fluidic connector **701** from one another.

Still referring to FIG. 8, undercut portions **771** are provided by the body **770** as part of the port of the body **770.** The undercut portions **771** are adapted to receive and hold the projections **737** of the body **715** when the lower portion of the body **715** is inserted into the port. The undercut portions **771** retain the projections **737** and keep the fluidic connector **701** connected to the body **770** once the projections **737** have been inserted sufficiently far enough into the port of the body **770.** The spring **745** is selected so that it exerts an outward force on the faces **741** and **743** of the body **715** and thus also exerts an outward force on the projections **737** of the body **715,** thereby retaining the projections **737** in place and retained by the undercut portions **771** of the port and thereby maintaining the fluidic connector **701** in a sealed connection with body **770.**

By pressing faces **741** and **743** inwardly and inserting the bottom portion of assembly 701 into a port of the body **770,** then releasing the pressure on faces **741** and **743,** an operator can obtain a connection that is sealed and leak-proof, and provides sufficient force to keep the sealed connection (even when a fluid under high pressures, such as from about 200 psi to 5000 psi or so is flowing through the tubing **705**) in place. An operator can make a desired connection in a LC or other analytical instrument system using the assembly **701** without the need for any torque, such as on a nut, without the need for a threaded connection, and without the need for any additional tools, such as a wrench or the like. Moreover, an operator can disconnect the assembly **701** from a port in the body **770** by pressing faces **741** and **743** inwardly and pulling on the body **715** or other portion of the assembly **701** and easily removing the assembly **701** from the port of the body **770,** again without the needed for any torque, without the need for disconnecting a threaded engagement (such as if a nut with threads is used), and without the need for any additional tools.

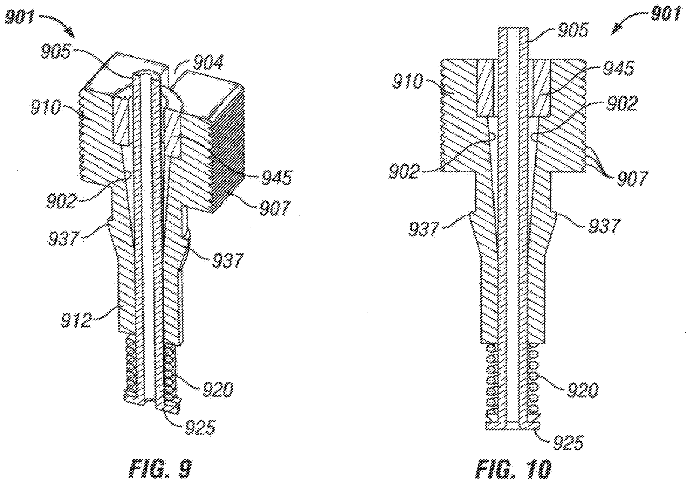

Referring now to FIGs. 9 and 10, another embodiment of a fitting connector assembly is shown in a cut-away view. In FIG. 9, the fluidic connector **901** includes a body having a head **910** at one end and a lower portion **912** at an opposing end. As seen in FIG. 9, a tube **905** extends from above the head **910** and through a passageway extending through the body. The tubing **905** has a flange **925** at its bottom end, which extends out from the lower portion **912** of the body. As shown in FIG. 9, the head **910** can be generally rectangular in shape (although head **910** can generally be of other shapes, such as circular, elliptical, and the like). A slot **904** is shown on one side of the head **910;** a similar and opposing slot (not shown in FIG. 9) would be on the opposing side in this particular embodiment, thus providing a generally rectangular head **910** with four sides, two opposing sides of which have slots therein. The fluidic connector **901** also includes a spring member **945** located at or near the top end of the body **910.** The head **910** also has splines **907,** which are provided to allow an operator to grasp and manipulate the fluidic connector **901** easily. The fluidic connector **901** has an interior portion, which includes a tapered portion **902,** which is generally conical in shape. As shown in FIG. 9, located below the head **910** are projections **939** extending out from the longitudinal axis of the fluidic connector **901.** Located below the lower portion **912** of the body is a spring **920,** which in this particular embodiment can be a coiled spring. FIG. 10 provides a cross-sectional view of the embodiment of the fluidic connector **901** shown in FIG. 9.

An operator can easily assembly the fluidic connector **901** by placing the tubing **905** through the spring **920,** through the central passageway through the lower portion **912** and head **910** of the body, and through the central passageway through the spring member **945,** and extending from the top end of the fluidic connector **901.** The spring member **945** can be removably secured within the head **910** if desired, or can be permanently attached or secured to the head **910.** The spring member **945** can be any one of number of different types of spring members, such as an elastomeric spring. The spring member **945** serves to push or bias the four sides of the head **910** outward from the longitudinal axis of the fluidic connector **901.** However, the spring member **945** can be selected (such as by selecting its shape, size, and material composition) so that an operator can easily push the two opposing sides of the head **910** which do not have slots **904** towards the longitudinal axis. The slots **904** allow the other two sides to move towards each other, and thus the projections **937** move towards each other and the longitudinal axis, thereby providing a smaller outer diameter than when in a resting or normal position (such as when no additional force is applied to opposing sides of head **910**). By squeezing the two opposing sides of head **910** which do not have slots **904,** the operator can move the projections closer together for easy insertion of the lower portion of the fluidic connector **901** into a port and, when the operator releases the two opposing sides, the projections **937** move outwardly from the longitudinal axis of the fluidic connector **901** and, due to their now greater outer diameter, can be securely held in place in the port. To remove the fluidic connector **901** once secured to a port, the operator can squeeze the two opposing sides of head **910** which do not have slots **904** towards one another and towards the longitudinal axis, thereby moving the projections **939** towards the longitudinal axis and reducing the outer diameter of the two projections **937,** at which point the operator can easily pull the entire (and still assembled) fluidic connector **901** from the port. The fluidic connector **901** thus allows an operator to quickly connect the fluidic connector **901** to a port, to quickly disconnect the fluidic connector **901** from a port, all without requiring the use of any tools or applying a particular force beyond that necessary to insert the fluidic connector **901** into the port as described. Moreover, the spring **920** pushes the flange **925** against the bottom of a port and thereby ensures a sealing connection when the fluidic connector **901** is connected to a port as just described.

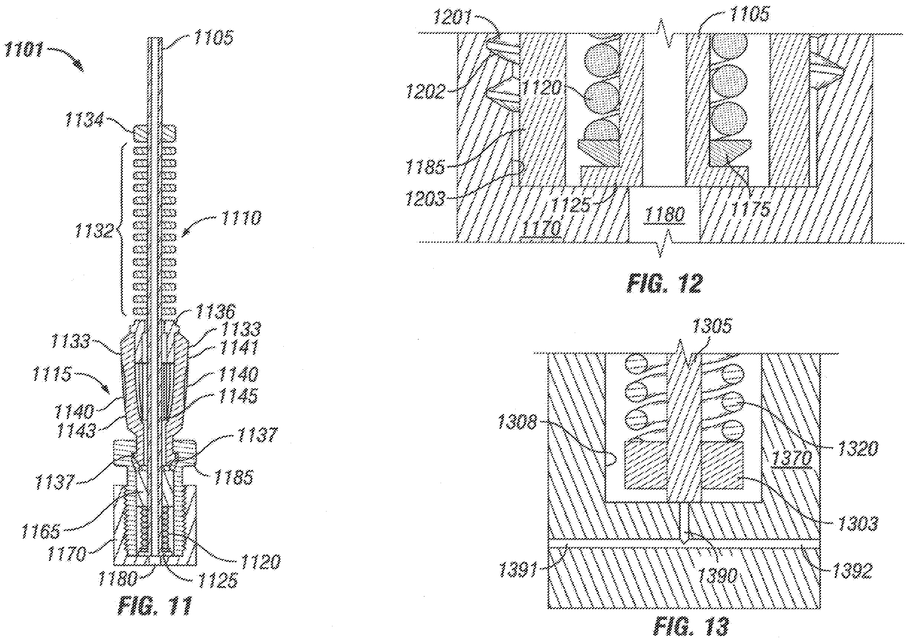

Referring now to FIG. 11, another embodiment of a fluidic connector **1101** is shown. In FIG. 11, the fluidic connector **1101** includes an extension portion **1110** and a body **1115.** The extension **1110** includes a top member **1134** and a series of toroidal members **1132.** Also included as a part of extension **1110** (but not shown in FIG. 11) is a spine member. The base **1136** of the extension **1110** is located within a portion of the body **1115.** The body **115** includes two opposing faces **1141** and **1143,** each of which has an indentation **1140.** In addition, each of the opposing faces **1141** and **1143** has a hinge **1133** to allow at least a portion of the face **1141** and **1143** to move inwardly and outwardly with respect to the longitudinal axis of the fluidic connector **1101.** The fluidic connector **1101** also includes a spring **1145** located within an interior portion of the body **1115.** The body **1115** also includes a lower portion which has projections **1137** adapted for removable engagement as described below. Located below the body **1115** is a ring member **1165,** and below the ring member **1165** is a spring **1120,** which in this particular embodiment and as shown in FIG. 11 is a coiled spring. As can be seen from FIG. 11, the tubing **1105** has a flange **1125** at one end, and the tubing **1105** extends along the longitudinal axis of the fluidic connector **1101** through a central open portion of the spring **1120** and through passageways in the ring **1165,** the body **1115,** the base **1136,** and the extension **1110.**

In FIG. 11, the fluidic connector **1101** is connected via an adapter **1185** to a port in a component or fitting **1170,** such as a component or fitting **1170** of the type used in an AI system. As shown in FIG. 11, the projections **1137** have an outer diameter greater than the top opening of the adapter **1185.** The undercut portions of the adapter **1185** hold the projections **1137** securely in place relative to the adapter **1187** once they are inserted into the adapter **1187.** As also shown in FIG. 11, the adapter **1187** has a portion which is externally threaded in this embodiment. Moreover, the port of component **1170** is internally threaded, and the external threaded portion of the adapter **1187** is selected and adapted so that adapter **1187** can be screwed into the port of the component **1170** and thereby securely and removably held in place and connected to the component **1170.** As the adapter **1187** is screwed into the port of component **1170,** the spring **1120** in the fluidic connector **1101** will be compressed and will exert a force upon the ring **1175,** which in turn will exert a force on the flange **25** and urge or press the flange **25** against the bottom of the port of component **1170** as shown in FIG. 11.

Referring now for FIG. 12, an enlarged cross-sectional view of a portion of the connected fluidic connector **1101** shown in FIG. 11 is provided. Like numerals are used for the same features and components shown in both FIGs. 11 and 12, although additional numerals are used for certain details better illustrated in FIG. 12. As shown in FIG. 12, the tubing **1105** extends through a spring **1120,** and both tubing **1105** and spring **1120** are located within a passageway in an adapter **1185.** The tubing **1105** has a flange **1125** at a bottom end, which is pressed against the bottom of the port of the component **1170.** As shown in FIG. 12, a fluid passageway **1180** extends from the bottom of the port of component **1170.** The fluidic connector **1101** in FIG. 12 also shows the backup ring **1175** in more detail. The backup ring **1175** is located between the bottom of the spring **1120** and the top of the flange **1125.** The backup ring **1175** at its bottom end or side has a smaller diameter than at its top end or side. The backup ring **1175** can be used to concentrate the force exerted on the flange **1125** to a smaller or reduced area of the surface of the flange **1125,** which has the advantage of keeping the fluid pressure area as small as possible during operation when a fluid is flowing under pressure through tubing **1105.** The backup ring **1175** thus helps maintain a sealed connection between the fluidic connector **1101** and the component **1170.** As also shown in FIG. 12, the port of the component **1170** has a lower portion **1203** and, above that, an internally threaded portion with internal threads **1202.** As noted above, the adapter **1185** has an externally threaded portion with external threads **1201.** The external threads **1201** are selected so that they can be removably and securely engaged with the internal threads **1202** of the port of component **1170.**

Referring now to FIG. 13, another embodiment of a fluidic connector is provided. As shown in FIG. 13, a component **1370** has a tee connection among fluid passageways **1390, 1391,** and **1392.** In this particular embodiment, the tube **1305** of the fluidic connector does not have a passageway therethrough, but is solid. Alternatively, the tube **1305** could be made of a semipermeable material to allow some fluid to flow therethrough, but only under pressure. As shown in FIG. 13, a bottom end of the tube **1305** is located adjacent to the bottom of the port **1308** of the component **1370** in the connected mode. Also shown in FIG. 13 is a bottom sleeve **1303.** The sleeve **1303** in this embodiment is attached to the tube **1305.** The sleeve **1303** can be attached to the tube **1305** through techniques such as fusing, crimping or overmolding. The sleeve **1303** can be advantageous because it provides a surface which is biased against the bottom of the port by the spring 1320 and provides additional surface area to provide a seal.

In this particular embodiment in FIG. 13, the fluidic connection acts as a pressure relief, such that when the pressure of a fluid in passageways **1390, 1391,** and **1392** exceeds a certain threshold amount, the tube **1305** will be pushed away from a sealing engagement with the bottom of the port and fluid will flow through passageway **1390** and into the port **1308.** Those skilled in the art will appreciate that the force exerted on the ring **1303** by the spring **1320** can be selected by selecting the shape, size and material composition of the spring **1320.**

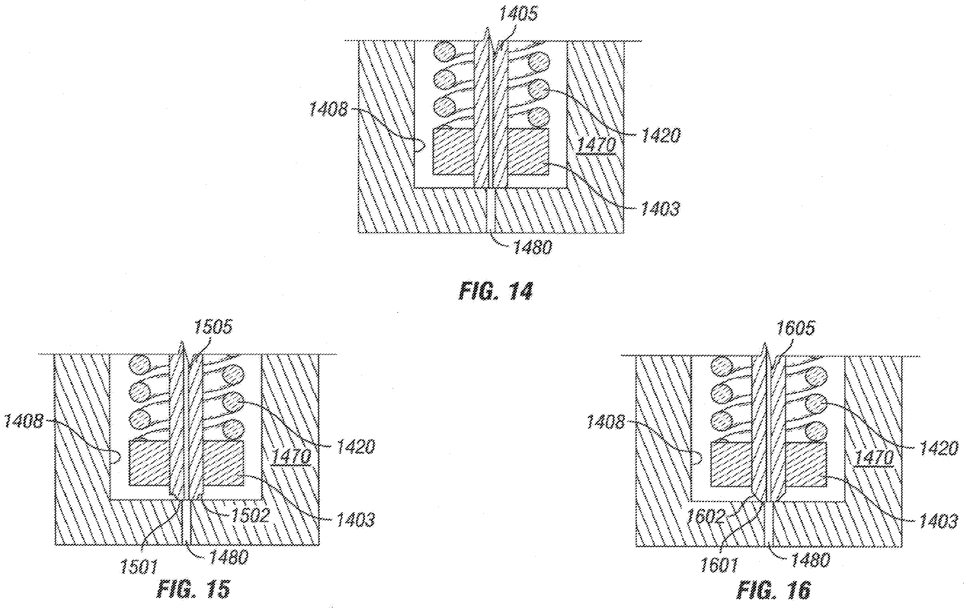

Referring now to FIGs. 14, 15, and 16, enlarged cross-sectional views are provided with respect to additional details of alternative embodiments of a fluidic connector in accordance with the present disclosure. Like features and components have the same numerals in FIGs. 14, 15, and 16 for convenience only; it will be apparent that each of FIGs. 14, 15, and 16 illustrates a different potential embodiment in accordance with the present disclosure.

Referring first to FIG. 14, a portion of a port 1408 in a component 1470 is shown. Located within the port **1408** is the bottom portion of a fluidic connector. In FIG. 14, the bottom end of tube **1405** is adjacent to the bottom of the port **1408** and sealingly engaged therewith. The fluidic connector includes a spring **1420** located above a sleeve **1403.** As shown in FIG. 14, a passageway for fluid communication extends through the tube **1405** and is aligned with the passageway **1480** in the component **1470.** As shown in FIG. 14, the bottom end of the tube **1405** in a sealing engagement with the port **1408** is an essentially or substantially flat surface.

Now referring to FIG. 15, a view of an embodiment similar to that shown in FIG. 14 is provided. In FIG. 15, a tube **1505** extends through the spring **1420** and sleeve **1403** and the fluidic connector is located and connected to a port **1408** in a component **1470.** The passageway in tube **1505** is aligned with the passageway **1480** in component **1470.** Instead of a flat surface at the bottom of the tube **1505,** however, a portion of the tube with a smaller diameter (and thus a smaller or reduced surface area) is in contact with the bottom of the port **1408.** The tube **1505** has a reduced diameter portion **1501** at its bottom end, and a tapering portion **1502** slightly above the reduced diameter portion **1501.** The use of a reduced diameter portion **1501** is helpful in concentrating the pressure load in a smaller area and thereby increases the pressure at the seal area. This has the advantage of allowing the fluidic connector to remain sealed when connected even at higher fluid pressures.

Another embodiment is shown in FIG. 16. In FIG. 16, a tube **1605** is connected via a fluidic connector in a port **1408** in a component **1470.** The passageway through the tube **1605** is aligned with the passageway **1480** in the component **1470.** As also shown in FIG. 16, a sleeve **1403** is located above the bottom end of the tube **1605** and below the spring **1420.** In FIG. 16, the bottom face of the tube **1605** has a reduced diameter and smaller surface area **1601,** with this reduced area **1601** providing the sealing engagement with the bottom of the port **1408.** The tube **1605** has a tapered portion **1602** between the reduced area portion **1601** and the portion of tube **1605** above it. As with the embodiment shown in FIG. 15, the embodiment shown in FIG. 16 provides the advantage of concentrating the load in a reduced area and thereby allows operation with a sealed connection at higher pressures.

Referring now to FIGs. 17-20, various views are provided of another embodiment of the present disclosure. In FIG. 17, a cross-sectional view of a connection assembly **1701** as connected to a port in a body **1770** is provided. As shown in FIG. 17, the assembly **1701** has tubing **1705** extending along a longitudinal axis of the assembly **1701,** an extension member **1710** having a top portion **1734,** a series of toroidal members **1732,** and a base **1736.** It will be appreciated that these various features correspond to the extension member **10,** toroidal members **32,** top member **34,** and base **36** described above and the foregoing description of such corresponding features applies equally. In FIG. 17, a bottom portion of the assembly **1701** is located in a port of a body **1770,** with the bottom portion including a ring member **1765,** a spring **1720,** a tip member **1750,** and a ring **1755.** As shown in FIG. 17, one end of the tubing **1705** is adjacent to and abutting the bottom of the port, and portions of the tip member **1750** surrounding the tubing **1705** are also adjacent to and abutting the bottom of the port of the body **1770.**

An enlarged cross-sectional view of the bottom portion of the assembly **1701** shown in FIG. 17 is provided in FIG. 18. In FIG. 18, the tip member **1755,** the tubing **1705,** and the ring **1755** are shown near the bottom of the port of the body **1770.** Those skilled in the art will appreciate that, as shown in FIG. 18, when the assembly **1701** is assembled and connected to a port in a body **1770,** the spring **1720** urges the tops of the ring **1755** and tip **1750** towards the bottom of the port of the body **1770,** thus providing a seal with sufficient force to prevent any leaks and prevent the tubing **1705** from being pushed out of the assembly **1701,** even when fluids at high pressures, such as from about 200 psi or so to about 5000 psi or so, are flowing through the tubing **1705** and the assembly **1701** as connected to the port of the body **1770** as shown in FIG. 18. In addition, it will be appreciated that the spring **1720** presses against and exerts an upward force on the ring member **1765,** thereby urging the bottom of the body **1715** and the projections near the bottom of the body **1715** (not shown in FIG. 18) against the top of the port of the body **1770.** FIG. 19 provides an additional view of the connection assembly shown in FIGs. 17 and 18, with the assembly shown in a sealing engagement, and with like features having the same numbers for convenience of the reader.

Referring now to FIG. 20, additional details are shown in the enlarged cross-sectional view of the fitting assembly **1701** as connected to a port in a body **1770.** As shown in FIG. 20, the tip **1750** has a first end and a top end, wherein the top end has a portion **1752** which has a smaller diameter than the bottom portion **1751.** As shown in FIG. 20, the bottom portion **1751** provides a greater area for contact with the bottom of the port of the body **1770** (although such contact is not shown in FIG. 20). As also shown in FIG. 20, the ring **1755** has a top end and a bottom end, wherein the top end has a portion **1756** with an exposed surface area greater than the surface area of the bottom end **1757** of the ring **1755.** As can be seen in FIG. 20, each of the ring **1755** and the tip **1750** have tapered portions between their respective top and bottom ends as shown in FIG. 20. In the case of the ring **1755,** the internal diameter defines a tapered portion, with the internal diameter at the bottom of the ring **1755** greater than the internal diameter of the ring **1755** at its top. In the case of the tip **1750,** the outside diameter of the tip **1750** defines a tapered portion, with the bottom of the tapered portion of the tip **1750** having a greater outside diameter than the top of the tapered portion of the tip **1750,** all as shown in FIG. 20. It will be appreciated that, when the assembly **1701** is connected to the port of the body **1770** and the spring **1720** exerts a force urging the ring **1755** and the tip **1750** against the bottom of the port of the body **1770,** the bottom end **1757** of the ring **1755** will be urged towards the longitudinal axis of the assembly **1701** and exert a force inwardly on the tip **1750** to prevent any leaks between the tubing **1705** and the tip **1750.**

The ring **1755** can be made of any one or more of the following materials, as may be desired for the anticipated use(s) of the assembly **1701:** PEEK, PEAK, PEKK, PEI, and/or PPSU. The tip **1750** can be made of any of the following materials, as may be desired for the anticipated use(s) of the assembly **1701:** polyetheretherketone (PEEK), polyaryletherketone (PAEK), polyetherketoneketone (PEKK), fluorinated ethylene propylene (FEP), ethylene tetrafluoroethylene (ETFE), polytetrafluoroethylene (PTFE), perfluoroalkoxy (PFA, also called perfluoroalkoxyethylene), polychlorotrifluoroethylene (PCTFE), polymer-sheathed fused silica (such as PEEKSil), fused silica, or silica borite, and can further include a filler material, which can include fibers, such as carbon fibers, glass fibers, nanofibers, and/or metallic fiber, depending on the pressures and fluids involved. Those skilled in the art will appreciate that the ring **1755** and tip **1750** can be of any number of desired shapes and sizes, preferably so that the tapered portions of the ring **1755** and the tip **1750** are adapted to cooperate with one another when the assembly **1701** is connected to the port of the body **1770.**

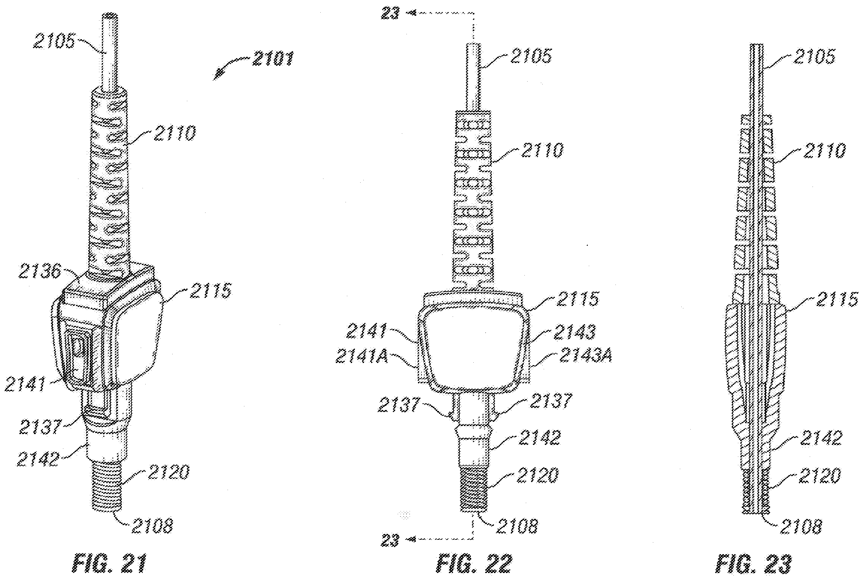

Referring now to FIGs. 21 - 25, another alternative embodiment of the connection assembly in accordance with the present disclosure is provided. As shown in FIG. 21, an assembly **2101** includes an extension member **2110,** tubing **2105,** a body **2115,** a spring **2120,** and a flange **2108** at one end of the tubing **2105.** From the prior discussion, it will be apparent that the fitting assembly **2101** is adapted to engage with a flat-bottomed port, such as in a component in an AI system (not shown in FIG. 21). The extension member **2110** in this particular embodiment is attached to the top **2136** of the body **2115.** As shown in FIG. 21, the body **2115** has four sides, which can be considered a front face, an opposing back face, and a side **2141** and an opposing side **2143** (not shown in FIG. 21). As shown in FIG. 21, the side **2141** has a projection **2137,** which is adapted to engage with a port and removably hold the assembly **2101** in a sealing connection with a port.

Now referring to FIGs. 22 and 23, alternative views of the assembly **2101** are provided. FIG. 22 provides a view of one face of the assembly **2101** (which for discussion purposes can be referred to as the "front" face), and FIG. 23 provides a cross-sectional view of the assembly **2101** taken along line E-E of FIG. 22. It will be appreciated that the same numbers are used to indicate the same features in FIGs. 21-25 for ease of reference by the reader. As shown in FIG. 22, the body **2115** has two opposing sides **2141** and **2143,** each of which has projections **2141a** and **2143a,** respectively. In addition, each of the two sides **2141** and **2143** has lower portions which have projections **2137** on each side of the body **2115.** In the assembly shown in FIG. 22, an operator can push on the projections **2141a** and **2143a** to push the two opposing sides **2141** and **2143** of the body **2115** inwardly towards the longitudinal axis of the assembly **2101,** thus moving projections **2137** inwardly as well and decreasing the length between the tips of the two opposing projections **2137.** Doing so allows the assembly **2101** to be quickly and easily inserted into, or removed from, a port (not shown in FIG. 22 or 23).

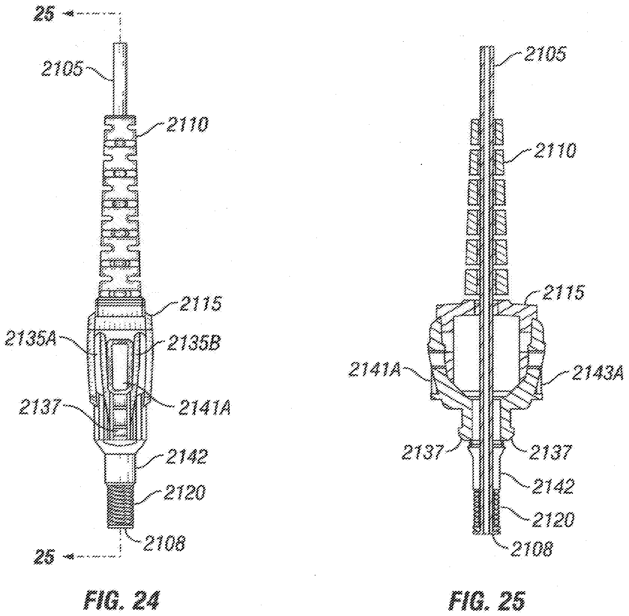

Referring now to FIGs. 24 and 25, additional alternative views of the assembly **2101** are provided. FIG. 24 provides a view of one side **2141** of the assembly **2101** (which for discussion purposes can be referred to as a "side" face), and FIG. 25 provides a cross-sectional view of the assembly **2101** taken along line F-F of FIG. 24. As noted, it will be appreciated that the same numbers are used to indicate the same features in FIGs. 21-25 for ease of reference by the reader. As shown in FIG. 24, the side **2141** of the body **2115** has two members **2135a** and **2135b** on either side of the projection **2141a.** Moreover, the projection **2141a** is shown to have a generally rectangular or parallelogram shape, with rounded corners. Those skilled in the art will appreciate that the shape of the projection **2141a** can easily be varied as may be desired. For example, the shape shown in FIGs. 21-25 provides a useful shape which is easily used by an operator to push sides **2141** and **2143** towards one another, but other shapes can likewise be used, such as ellipses, circles, squares, triangles, and so forth.

Among other features to note include the alternating grooves in the extension member **2110.** As indicated by a comparison of FIGs. 21-25, it can be seen that the grooves in extension member **2110** are essentially arcs which extend roughly 180 degrees around the generally conical edge of the extension member **2110.** Again, those skilled in the art will appreciate that differing patterns and configurations can be used for the extension member **2110.** The alternating grooves in extension **2110** provide a balance between the strength needed for the extension **2110** to provide effective stress relief for the tubing **2105,** while avoiding the use of too much material that is not needed. Those skilled in the art will appreciate that the features of the assembly **2101** as shown in FIGs. 21-25 can be made of the same materials as similar items and features in the embodiments previously described in this disclosure.

Additional embodiments of a quick connect/disconnect assembly are illustrated in Figs.26-31. It will be appreciated that like features and items will have the same numbering in Figs.26-31 for convenience. In Fig. 26, a cross-sectional view of an assembly **2601** is provided which includes a body **2602** which has a passageway therethrough in which tubing is located along the longitudinal axis of body **2602,** with the tubing having an inner tube **2605b** and an outer tube **2605a.** Also located within a first portion of the body **2602** (i.e., the "top" portion of body **2602** in Fig. 26) is a spring element **2620.** As illustrated in Fig. 26, the spring **2620** is a coiled spring which is located around the tubing and within the body **2620.** Also located within the body **2602** is a bearing element **2625.** As shown in Fig. 26, one side of the bearing element is located at the bottom end of and abuts the spring **2620.** The other side of the bearing **2625** is adjacent to and abuts a sleeve **2610** which surrounds a portion of the outer tubing **2605a** (and thus inner tubing **2605b** as well). The bottom portion of the body **2602** has inwardly projecting shoulders **2637,** shown on both sides of the body **2602** in Fig. 26, each having a face **2638.**

The assembly **2601** in Fig. 26 also includes a port **2670,** which can be a port for an AI system or for any component in an AI system. The port **2670** has an opening at one end through which the tubing **2605a** and **2605b** extends. The port **2670** also has a flat-bottomed port and a passageway **2675** which allows for fluid communication with a fluid in the interior of the inner tube **2605b.** The port **2670** also includes projections **2677** on both sides, each of which has a projection face **2678.** As shown in Fig. 26, the body **2602** and port **2670** are removably attached to each other and held together by the engagement of the projections **2677** of the port **2670** and the shoulders **2637** of the body **2602,** with the each of the faces **2678** abutting the corresponding faces **2638.** The assembly **2601** is thus shown in a connected configuration in Fig. 26.

The inner and outer tubing **2605a** and **2605b** are configured so that one end of the inner tubing **2605b** abuts against the bottom of the flat-bottomed port of port body **2670.** Also shown in Fig. 26 is a sleeve **2610,** which surrounds a portion of the outer tubing **2605a.** One end of the sleeve **2610** abuts one side of the bearing **2625,** and the other end of the sleeve **2610** has a recess therein in which a tip **2615** is located. The configuration and use of the tubing with sleeve **2610** and tip **2615** is described in more detail in co-pending U.S. non-provisional patent application Serial No. 14/992,041, filed October 23, 2015 and titled "Face-Sealing Fluidic Connection System," which is hereby incorporated by reference as if fully set forth herein. Those skilled in the art will appreciate and understand, however, that the present disclosure and its embodiments may such use other tubing configurations as may be desirable for a given application, and are not limited to those shown and described herein.

Those skilled in the art will appreciate that, depending on the intended application, including without limitation the expected fluidic pressures, the nature of the fluids to be used, the nature of the samples to be analyzed, and the like, the composition of the materials used, as well as the specific shapes and sizes, of the various components and features of the fluidic connector assembly **2601** as shown and described above can be varied. For example, in applications in which biocompatibility is desired, the tubing **2605a** and **2605b** can be made of the same or different materials, including for example [polyetheretherketone (PEEK), polyaryletherketone (PAEK), polyetherketoneketone (PEKK), fluorinated ethylene propylene (FEP), ethylene tetrafluoroethylene (ETFE), polytetrafluoroethylene (PTFE), perfluoroalkoxy (PFA, also called perfluoroalkoxyethylene), polychlorotrifluoroethylene (PCTFE), polymer-sheathed fused silica (such as PEEKSil), fused silica, or silica borite, and can further include a filler material, which can include fibers, such as carbon fibers, glass fibers, nanofibers, and/or metallic fibers, depending on the pressures and fluids involved. It is anticipated that the assembly **2601** will be of particular usefulness in applications in which biological materials are involved. In such situations, it is often desirable for the fluid path to contain only biocompatible materials. In the embodiment shown and described, this is achieved because the tubing **2605b,** the tip **2615** and the port body **2670** are the only components of the assembly **2601** touching the fluid, an even then tip **2615** need not necessarily come into contact with the fluid if the sealing connection between the end of the inner tubing **2605b** and the port body **2670** is made. In the embodiment shown in FIG. 26, then, the spring **2620,** as well as the body **2602,** the bearing **2625,** and the sleeve **2610** can be made of metal, such as stainless steel, steel, titanium, nickel, and/or nitinol. Alternatively, the spring **2620,** as well as the body **2602,** the bearing **2625,** and the sleeve **2610** can be made of metal, or can be made of PEEK, PEAK, PEKK, PEI, and/or PPSU.

A user or operator can quickly and easily connect the body **2602** and port **2670.** To connect the body **2602** and port **2670,** a user can insert one end of tubing **2605a** and **2605b** into and through the passageway through the longitudinal axis of body **2602,** through the sleeve **2610,** and into the opening of port **2610.** The user can then pinch the two tabs **2640** extending radially outwardly from the longitudinal axis of the body **2602,** and push the two tabs **2640** towards the longintudinal axis (i.e., radially inwardly). By doing so, the user thus moves the two inwardly projections **2637** of the body radially outwardly, and thereby increases the inner diameter between the two projections **2637.** Once the inner diameter between the two projections **2637** is greater than the outer diameter of the two shoulders or projections **2677** of the port **2670,** the bottom end of the body **2602** can easily and quickly fit over the projections **2677.** Once the projections **2637** are below and past the projections **2677,** the user can simply release the force on the two tabs **2640** and the projections **2637** of body **2602** will automatically move back radially inwardly, thereby engaging faces **2638** and **2678** so that the body **2602** and the port **2670** are held securely and removably together. To disconnect the body **2602** and port **2670** from one another, a user can simply urge the tabs **2640** radially inwardly and towards the longitudinal axis of the assembly **2601,** thereby moving the projections **2637** of the body **2602** radially outward and disengaging the projections **2637** and **2677.** Once the inner diameter defined by the two projections **2637** is greater than the outer diameter defined by the projections **2677** of the port **2670,** the body **2602** can be pulled away and removed from the port **2670** quickly and easily. As shown in Fig. 26, the assembly **2601** is in a connected configuration.

In this particular embodiment, the two tabs **2640** and the bottom portion of the body **2602** can be substantially rectangular in shape. Those skilled in the art will appreciate, however, that the tabs **2640** and/or bottom portion of the body **2602** can be curved and describe an arc around the longitudinal axis, or can even be circular in shape and extend all the way around the longitudinal axis of the assembly **2601** if desired.

Those skilled in the art will appreciate that, when making a connection, a user will also need to push the body **2602** towards the port **2670** and along the longitudinal axis of the assembly **2601,** and doing so will thereby compress the spring **2620.** As the spring **2620** is compressed, it will exert a force longitudinally against the bearing **2625** and thereby against the sleeve **2610,** which in turn exerts a force against the tip **2615,** urging it against the bottom face **2673** of the port **2670** and sealing the inner tubing **2605b** in the port to provide a leak-free seal. The spring **2620** can be chosen so that its size and compressive force is such that it fits within the body **2602** as shown and also is easily compressed by a user by hand without the need for tools or fixtures, yet will continue to exert a compressive force against the bearing **2625** to keep the tip **2615** abutting the bottom of the port and thus keep the leak-free seal while the assembly **2601** remains in a connected configuration. We believe that, with the appropriate selection of materials and spring size, a leak-free seal of capillary tubing can be obtained in a flat-bottomed port with fluid flowing through the tubing at pressures of up to 20,000 psi without any leakage or extrusion of the tubing from the assembly **2601.**

Referring now to Fig. 27, another cross-sectional view of the assembly **2601** is provided. It will be appreciated that in Fig. 27, the body **2602,** tubing **2605a** and **2605b,** spring **2620,** and bearing **2625,** as well as sleeve **2610** and tip **2615** can be the same as shown in Fig. 26 and as described above. In Fig. 27, the port **2670'** differs in that it no longer provides a conventional flat-bottomed port (as was shown in Fig. 26 and described above). Instead, the bottom of the opening adapted to receive and sealingly hold the tubing in the port **2670'** has a standing boss portion **2672** which extends upwardly from the bottom of the opening and radially inwardly towards the longitudinal axis of the port **2670',** thus providing a flat-bottomed face **2673'** which has a smaller diameter than the inner diameter of the opening of the port **2670'** into which the tubing is inserted and is received. As shown in Fig. 27, only the inner tube **2605b** is in contact with the face **2673'** of the port **2670'.** Those skilled in the art will appreciate, however, that the size of the face **2673'** of the port **2670'** can be chosen so that, when desired, some or all of the tip 2615 and inner tube **2605b** together abut the face **2673'** instead of just the tube **2605b.**

Conventional designs for ports and connections in most UHPLC and HPLC instruments and components typically have a 10-32 coned port with a flat bottom for creating the fluidic seal with one end of tubing connected therein. The standing boss **2672** provides a feature that allows for use of the assembly **2601** with ports like port **2670'** in applications with elevated pressures not common for conventional quick connections. Conventional 10-32 port bottoms typically have a sealing area of .0032 square inches, whereas the standing boss **2672** can be used to provide a port bottom face **2673'** with an area of .00031 square inches. The sealing area becomes important when considering the pressure applied to the end of the tubing. Assuming the tubing can be pressed by the user, via a fitting assembly **2601** or otherwise by conventional assemblies, to about 10 lbs of load, then the pressure at the tube end in a standard port is 3,125 psi vs. 32,258 in a port **2670'** with the standing boss **2672** and the reduced flat bottomed port face **2673'.** Those skilled in the art will appreciate that the standing boss **2672** can be provided in a number of ways; it can be machined directly in the port bottom or can be obtained via the use of an additional insert that fits in the port bottom, or in other ways.

Now referring to Fig. 28, a cross-sectional view of an embodiment of a connection assembly **2801** is provided. In Fig. 28, a body **2602** is provided, which may be the same as the body **2602** described above. The body **2602** has tubing **2605a** and **2605b** extending through the passageway through the body **2602,** and has a coil spring **2620** located within a top portion of the body **2602** and around the tubing, with one end of the coil spring **2620** abutting an interior top end of the body **2602** and the other end abutting one side of a bearing **2625.** The body **2602** further has two tabs **2640** and radially extending inward projections **2677.**

In Fig. 28, the assembly **2801** includes one end of a column **2855,** which has an exterior or outer layer **2850.** One end of the outer layer **2850** has external threads **2851,** which are adapted to securely engage with the internal threads **2861** of the adapter **2860.** As shown in Fig. 28, the adapter **2860** has a first end which provides a port adapted to securely and sealingly receive one end of tubing **2605a** and **2605b,** as well as securely and removably engage with the body **2602** via the projections **2877** on either side of the adapter **2860.** The other end of the adapter **2860** (i.e., the bottom end as illustrated in Fig. 28) has a recess therein which has an internally threaded portion **2851.** As shown in Fig. 28, a first portion of the recess in the bottom end of the adapter **2860** is adapted to removably engage with the outer layer **2850** and column **2855.**

The adapter **2860** also has a seat portion **2862** therein which, in the embodiment shown in Fig. 28, has an insert **2870** removably seated therein. As shown in Fig. 28, the insert **2870** has a first side which has standing boss **2872** which provides a face **2873** against which the inner tube **2605b** and at least a portion of the tip **2615** abut to provide a removably sealing engagement. The standing boss **1272** provides the same advantages as described above with respect to a smaller area for forming the seal with one end of the tubing. The second side of the insert **2870** has a recess portion, in which a filter element **2880** (such as a frit) is located. Those skilled in the art will appreciate that, although Fig. 28 illustrates the use of the body **2602** with an adapter **2860** for connecting to one end of a column **2855,** the adapter **2860** can be used with body **2602** to provide a quick and easy connect/disconnect function (such as described above) in connection with any one of a number of components in an LC or AI system.

Turning now to Fig. 29, a cross-sectional view of an assembly **2901** is illustrated to show another embodiment of this disclosure. The assembly **2901** includes a body **2602** through which one end or tubing **2605a** and **2605b** extends, together with a spring **2625** located within and along the longitudinal axis of the body **2602.** The body **2602** has tabs **2640** and radially inward projections **2637** as described above.

In the assembly **2901,** an adapter **2970** is used together with the body **2602** to connect the tubing **2605a** and **2605b** to a union **2910.** As illustrated in Fig. 29, the union **2910** may have a conventional port, such as one having an internally threaded portion with 10-32 threads, a conical taper, and then a flat-bottomed face for sealing against an end of a tube. As shown in Fig. 29, the adapter **2970** has a passageway therethrough along its longitudinal axis, with a first or top end as shown in Fig. 29 having proximal thereto radially outwardly extending shoulders or projections **2977** on either side, each of which have faces **2978** and are adapted to removably engage with the projections **2637** on either side of the body **2602.** In addition, the first or top end of the adapter **2970** has an opening therein which is adapted to receive one end of the tubing **2605a** and **2605b,** together with the sleeve **2610.**

The bottom end of the adapter **2970** has an externally threaded portion **2951,** which is adapted to removably engage with an internally threaded recess portion of the first or top end of the union **2910** shown in Fig. 29. As shown in Fig. 29, the union **2910** may have two ends, each having an internal port, each with an internally threaded portion, a conically tapered portion, and a flat-bottomed portion for receiving one end of a tube or, as shown in Fig. 29, a connector **2920.** The connector **2920** shown in Fig. 29 has two ends and a fluid passageway extending therethrough along its longitudinal axis. The first or top end of the connector **2920** has a portion which is of a size and shape that is adapted to removably fit within passageway **2972** of the adapter **2970.** The adapter **2970** has a neck portion **2921,** which has a narrower inner diameter than the portion of the passageway **2972** below the neck portion **2921,** as well as the portion of the passageway **2972** located above the neck portion **2921** as illustrated in Fig. 29. The first or top end of the connector **2920** extends through the passageway **2972** of the adapted **2970** and past the top end of the neck portion **2921.** As shown in Fig. 29, the first or top end of the connector **2920** abuts against the bottom end of the tip **2615** and the inner tube **2605b** to provide a removable seal, with the passageways in the inner tube **2605b** and the connector **2920** aligned to allow fluid to pass therethrough when the assembly **2901** is connected as is shown in Fig. 29.

A cross-sectional view of another alternative embodiment is provided in Fig. 30. A connection assembly **3001** (shown in a connected configuration) includes a first body **3002,** a second body **3003,** a spring element **3020,** a washer element **3025,** and a bearing element **3068,** each having a passageway therethrough along a longitudinal axis of the assembly **3001.** Located within the passageway of each of the first body **3002,** second body **3003,** spring **3020,** washer **3025,** and bearing **3068** is tubing **3005.** As shown in Fig. 30, one end of tubing **3005** extends through the passageway of the assembly **3001** and a portion of which is located in a first port at one end of a connector **3070.** Also shown in Fig. 30 is a tip **3015** and a sleeve **3010,** each of which surrounds a portion of the tubing **3005.**

The first body **3002** has arms or tabs **3040** on either side. It will be appreciated that the arms **3040** can be rectangular, arc-shaped, or circular and extend all the way around the longitudinal axis of the body **3002,** or may have such other shape as desired. The body **3002** also has inwardly radially projecting shoulders **3006** on either side. When a user presses the arms **3040** radially inwardly, the shoulders **3006** also move radially inwardly. When the shoulders **3006** move radially inwardly, they press inwardly against portions **3007** of the second body **3003.** When the portions **3007** of the second body **3003** are moved inwardly towards the longitudinal axis of the assembly **3001,** the projections **3077** at the second or bottom end of the second body **3003** also move radially inwardly and therefore define a narrower outer diameter proximal the second or bottom end of the second body **3003.** As shown in Fig. 30, the projections **3077** in a connected configuration are located and removably held within a recess **3040** proximal a first or top end of the connector **3070.** In addition, a bearing **3067** is located on the bottom side of the second body **3003** and a first or top side of the bearing **3067** abuts against the bottom end of the second body **3003.**

As also shown in Fig. 30, located within the passageway of the second body **3003** and proximal a first or top end thereof is a spring element **3020.** A top side of the spring **3020** abuts against an interior surface of the top end of the second body **3003,** while the bottom side of the spring **3020** abuts against a first or top side of the washer **3025.** The first or top side of the washer **3025** has shoulders adapted to receive and hold the bottom side of the spring **3020,** while the bottom side of the washer has shoulders adapted to receive and hole a first or top end of the sleeve **3010.**

The connector **3070** in Fig. 30 has at a top or first end an opening defined by radially inward projections **3071,** which define an inner diameter narrower than that of the recess **3040.** As also shown in Fig. 30, the connector **3070** has a flat-bottomed port at one end which is adapted to receive and sealingly hold one end of the tubing **3005,** tip **3015,** and sleeve **3010.** The connector **3070** further has a passageway **3075** from the flat-bottomed port to a second port, which as shown in Fig. 30, may be of a conventional type of port, with a flat bottom for receiving an end of tubing **3090** therein, a conically tapered portion for engaging with a ferrule **3091,** and an internally threaded portion adapted to removably engage with an externally threaded end of a nut **3092.**

Those skilled in the art will appreciate that, depending on the intended application, including without limitation the expected fluidic pressures, the nature of the fluids to be used, the nature of the samples to be analyzed, and the like, the composition of the materials used, as well as the specific shapes and sizes, of the various components and features of the fluidic connector assembly **3001** as shown and described above can be varied. For example, in applications in which biocompatibility is desired, the tubing **3005** can be made of the same or different materials, including for example [polyetheretherketone (PEEK), polyaryletherketone (PAEK), polyetherketoneketone (PEKK), fluorinated ethylene propylene (FEP), ethylene tetrafluoroethylene (ETFE), polytetrafluoroethylene (PTFE), perfluoroalkoxy (PFA, also called perfluoroalkoxyethylene), polychlorotrifluoroethylene (PCTFE), polymer-sheathed fused silica (such as PEEKSil), fused silica, or silica borite, and can further include a filler material, which can include fibers, such as carbon fibers, glass fibers, nanofibers, and/or metallic fibers, depending on the pressures and fluids involved. It is anticipated that the assembly **2601** will be of particular usefulness in applications in which biological materials are involved. In such situations, it is often desirable for the fluid path to contain only biocompatible materials. In the embodiment shown and described, this is achieved because the tubing **3005,** the tip **3015** and the port body **3070** are the only components of the assembly **3001** touching the fluid, and even then tip **3015** need not necessarily come into contact with the fluid if the sealing connection between the end of the tubing **3005** and the port body **3070** is made.

In the embodiment shown in FIG. 30, then, the spring **3020,** as well as the first body **3002,** the second body **3003,** the bearing **3025,** the washer **3068,** and the sleeve **3010** can be made of metal, such as stainless steel, steel, titanium, nickel, and/or nitinol. Alternatively, the spring **3020,** as well as the first body **3002,** the second body **3003,** the bearing **3025,** the washer **3068,** and the sleeve **3010** can be made of one or more non-metals, such as PEEK, PEAK, PEKK, PEI, and/or PPSU.

A user or operator can quickly and easily connect the assembly **3001.** To connect the first body **3002** and second body **3003** with the connector **3070,** a user can insert one end of tubing **3005** into and through the passageway through the longitudinal axis of first body **3002,** second body **3003,** spring **3020,** washer **3025,** bearing **3068,** and through the sleeve **3010,** and into the opening of connector **3079.** The user can then pinch the two tabs **3040** extending radially inwardly towards the longitudinal axis of the body **2602,** which then pushes the two shoulders **3006** towards the longitudinal axis (i.e., radially inwardly). By doing so, the user thus moves the two projections **3077** of the second body **3003** radially inwardly, and thereby decreases the outer diameter of the bottom end of the second body **3003** as defined by the two projections **3077.** Once the outer diameter defined by the two projections **3077** is less than the inner diameter of the two shoulders or projections **3071** at the top end of the connector **3070,** the bottom end of the second body **3003** can easily and quickly fit past the projections **3071** and into the recess at the top end of the connector **3070.** Once the projections **3077** are below and past the projections **3071,** the user can simply release the force on the two tabs **3040** and the projections **3077** of the second body **3003** will automatically move back radially outwardly, thereby engaging the projections **3071** and **3077** so that the bottom end of the second body **3003** and the top end of the connector **3070** are held securely and removably together. To disconnect the second body **3003** and the connector **3070** from one another, a user can simply urge the tabs **3040** radially inwardly and towards the longitudinal axis of the assembly **3001,** thereby moving the projections **3077** of the second body **3003** radially inward and disengaging the projections **3077** and **3071.** Once the outer diameter defined by the two projections **3077** is less than the inner diameter defined by the projections **3071** of the connector **3070,** the bottom end of the second body **3003** can be pulled away and removed from the connector **3070** quickly and easily.

Those skilled in the art will appreciate that, when making a connection, a user will also need to push the first body **3002,** and thereby the second body **3003,** longitudinally towards the connector **3070** and along the longitudinal axis of the assembly **3001.** Doing so will thereby compress the spring **3020.** As the spring **3020** is compressed, it will exert a force longitudinally against the washer **3025** and thereby against the sleeve **3010,** which in turn exerts a force against the tip **3015,** urging it against the bottom face of the port at the top end of the connector **3070** and sealing the tubing **3005** in the port of the connector **3070** to provide a leak-free seal. It will be appreciated that the spring **3020** can be selected so that it is strong enough to continue to exert enough force on the sleeve **3010** and thereby tip **3015** as long as the assembly **3001** remains in a connected configuration, yet will be of such strength that a user will be able to compress the spring **3020** when making a connection with assembly **3001** by hand and without the need for any tools or fixtures. We believe that, with the appropriate selection of materials and spring size, a leak-free seal of capillary tubing can be obtained in a flat-bottomed port with fluid flowing through the tubing at pressures of up to 20,000 psi without any leakage or extrusion of the tubing from the assembly **3001.**

Referring now to Fig. 31, a cross-sectional view of another embodiment of a connection assembly is provided. In Fig. 31, the assembly **3101** includes a first body **3002,** a second body **3003,** a spring **3020,** a washer **3025,** tubing **3005,** a tip **3015,** a bearing **3068,** and a sleeve **3010,** each of which may be configured like that described above, and each of which has passageways therethrough. It will be appreciated that the assembly **3101** is shown in a connected configuration.

In Fig. 31, the assembly **3101** includes an adapter **3170,** which can be used to connect one end of the tubing **3005** to a first end of a column **3195.** The adapter **3170** has a first or top end which has radially inward projections **3171,** which are adapted to removably engage with the projections **3077** of the second body **3003.** As shown in Fig. 31, in a connected configuration, the inner diameter defined by the projections **3171** of the adapter **3170** is less than the outer diameter defined by the projections **3077,** so the bottom end of the second body **3003** is held within the first or top end of the adapter **3170.** The adapter **3170** also includes an interior recess **3173** extending along a portion of its longitudinal axis, with the recess **3173** of a shape and size and configured to receive and removably hold therein the sleeve **3010,** tip **3015,** and tubing **3005.** The bottom end of the recess **3173** defines a flat-bottom face.

An insert **3180** can be located at the top end of the recess defined at the second or bottom end of the adapter **3170.** As illustrated in Fig. 31, the insert **3180** has a first or top face and a second or bottom face. Because the top end of the insert **3180** has a standing boss **3182,** the top face **3183** of the insert **3180** defines a smaller surface area than that of the port or recess **3173.** The smaller surface area of face **3183** provides the same advantages as described above by concentrating the pressure load applied by a user to connect the assembly **3101** and thereby allow the assembly **3101** to operate leak-free at higher pressures.

Located in a recess on the bottom side of the insert **3180** is a filter element **3190,** which can be a frit of a conventional type. As shown in Fig. 31, the outer layer **3197** of the column **3195** is secured to the bottom end of the adapter **3170** by the mating of the external threads proximal the top or first end of the outer layer **3197** and the internal threads proximal the second or bottom end of the adapter **3170.**

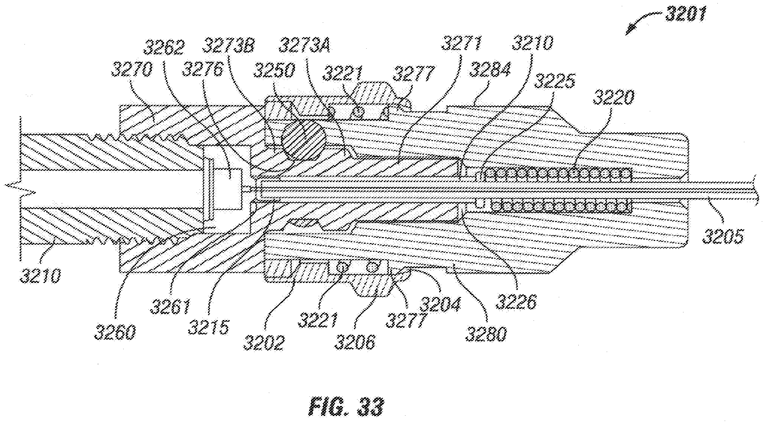

Referring now to Figs. 32and 33, an alternative embodiment of a quick connect/disconnect assembly is illustrated. Fig. 32 is an exploded isometric illustration of an embodiment of an assembly in accordance with the present disclosure in a disconnected configuration, while Fig. 33 is a cross-sectional view of the assembly of Fig. 32 in a connected configuration. In Fig. 32, the assembly **3201** provides an alternative embodiment for a connection assembly which may be quickly and easily connected or disconnected by an operator or user by hand with an axial force and without any requirement for any torque forces, tools, or other equipment. The assembly **3201** includes a column **3210** which has attached by threaded engagement at one end thereof an adaptor **3270,** with the adaptor **3270** having a projecting end portion **3271.** Also shown in Fig. 32 is a portion of tubing **3205,** which has at one end a tip **3215.** A portion of the tubing **3205** extends through and along the longitudinal axis of a connector **3280.** Also shown in Fig. 32 is a collar **3202,** which is proximal one end of the connector **3280** and extends around an exterior portion of the connector **3280** at that end. It will be appreciated that the column **3210,** adaptor **3270,** connector **3280,** and collar **3202** can be made of a variety of materials, including stainless steel, steel, titanium, nickel, PEEK, PEAK, PEKK, PEI, and/or PPSU.

As shown in Fig. 33, the assembly **3201** is illustrated in a cross-sectional view in a connected configuration. The adaptor **3270** has proximal one end an interior threaded portion adapted to removably engage with an exterior threaded portion proximal one end of the column **3210.** As shown in Fig. 33, a frit or filter **3262** is located in a holder **3260,** with one side of the holder **3260** abutting against one end of the column **3210.** In addition, the holder **3262** has a tapered portion **3261** on the side opposite the column **3210,** with this latter side adapted to form a sealing engagement with the tip **3215** at one end of the tubing **3205.** The tapered portion **3215** can be a frusto-conical shape or any other shape that provides a reduced diameter surface at the side of the holder **3260** adapted to form a seal with the end of the tubing **3205** and/or tip **3215** (as shown in this particular embodiment of Fig. 33).

The adapter **3270** has an end portion **3271** proximal the second end of the adapter **3270**, which is located in a recess located within and proximal to a first end of the connector **3280**. As shown in Fig. 33, the adapter **3270** has a passageway therethrough, portions of which are adapted to receive and hold the tubing **3205**, a tubing sleeve **3210**, as well as the holder **3260** and the first end of the column **3270**. In addition, the adapter **3270** has a reduced outer diameter portion **3276** and, on either side thereof, has shoulder (or enlarged diameter) portions **3273a** and **3273b**. As shown in Fig. 33, a ball **3250** is located between the shoulder portions **3273a** and **3273b** and rests against the exterior or the adapter **3270** at the reduced diameter portion **3276**. The ball **3250** and the adapter **3270** thus form a ball detent connection to hold the collar **3202** and connector **3280** in place once the ball **3250** is located between the two shoulder portions **3273a** and **3273b**.

Still referring to Fig. 33, a spring **3221** is located in a gap area located between the interior surface of a portion of the collar **3202** and an exterior surface portion of the connector **3280**, with one end of the spring **3221** abutting against a shoulder or radially outward projecting portion **3277** of the connector **3280**. The spring **3221** serves to bias or push the connector **3280** and collar **3202** away from one another, thereby keeping the collar **3202** in place proximal one end of the connector **3280** unless and until an operator or user pushes the collar **3202** away from the column and towards the right in Fig. 33. Once an operator so pushes the collar **3202** with enough force, the collar **3202** and ball **3250** will move to the right of the shoulder **3273a**, and at that point the adapter **3270** can be quickly and easily removed from the connector **3280** by simply pulling the two apart. The collar **3202** includes an enlarged diameter portion, or shoulder portion, **3206** in order to allow an operator to easily grasp or push or pull the collar **3202** in whatever direction is desired.

To connect the adapter **3270** and connector **3280** when they are not yet connected, an operator can easily and quickly move the collar **3202** away from the left end of the connector **3280** and towards the right end, thereby moving the ball **3250** up and out of the reduced diameter portion **3276**, then insert the first end portion **3271** of the adapter **3270** into the recess in the end of the connector **3280**. The operator can then push the collar **3202** back towards the left (in Fig. 33) and the ball **3250** will engage and be located within the shoulders **3273a** and **3273b**. The ball **3250** and detent configuration and the spring **3221** will hold the collar **3202** in place once an operator has made a connection. At one end of the collar **3202** are radially inwardly projecting shoulders **3204**. As shown in Fig. 33, the shoulder **3204** have an interior diameter which is less than that of the shoulders **3277** of the connector **3280**, and less than that of the portion **3284** of the connector **3280**. The shoulders **3277** and **3284** thus prevent the collar **3202** from moving either left or right by more than a specific amount. By selecting the distance between the shoulders **3277** and **3284**, and by selecting the spring **3221**, a preselected biasing force can be obtained to bias the collar **3202** and the connector **3280** towards a position in which the collar **3202** is located at or near the end of the connector **3280** which abuts or is proximal the adapter **3270**.

The connector **3280** shown in Fig. 33 has a second spring **3220** located therein. The spring **3220** is located in an interior passageway portion of the connector **3280**, with one end held in the passageway by the end of the connector **3280**, and the other end of the spring **3220** abutting one side of a washer **3225**. The other side of the washer **3225** abuts the sleeve 3210. The spring **3220** thus provides an axial force biasing the washer **3225** and therefore the sleeve **3210**, as well as the tubing **3205** and the tip **3215**, towards and against one side of the holder **3260**. As detailed above, the reduced surface area of the holder **3260** which is provided by the tapered portion **3261** allows a much greater force per unit of area to be obtained where the tip **315** abuts the holder **3261** given a particular axial load from the spring **3220**. The spring **3220** can be selected so that it provides a preselected force to bias or push the tip **3215** against the holder **3261** to obtain a sealing engagement. It will be appreciated that, depending on the intended application of the connection assembly **3201**, a higher or lower fluidic pressure may be used and so a higher or lower pressure may be desired to ensure that the tubing **3205** is not extruded from the assembly **3201** by the fluidic force and that there is no leakage of the fluid.

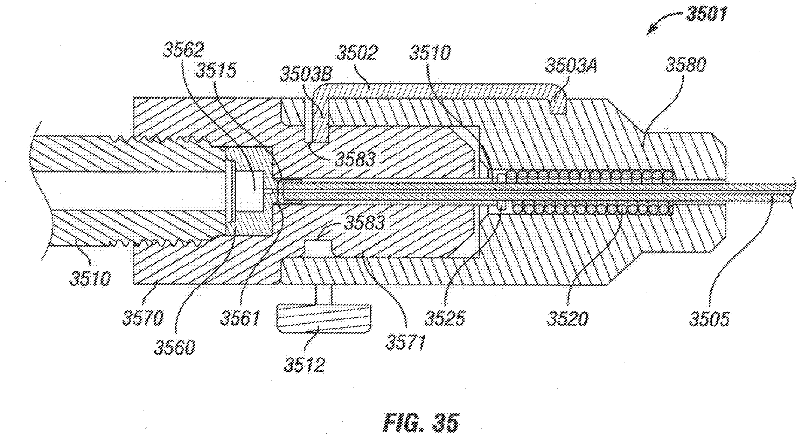

Referring now to Figs. 34 and 35, another embodiment of a connection assembly is provided. Fig. 34 is an exploded isometric view of the connection assembly **3501** in a disconnected configuration, while Fig. 35 is a cross-sectional view of the assembly **3501** in a connected configuration.

In Fig. 34, the assembly **3501** includes a column **3510**, an adapter **3570**, a collar **3502**, a connector **3580**, and tubing **3505**. As shown in Fig. 34, one end of the tubing has a tip **3515**. In addition, the collar **3502** has a first or front portion **3503b** and a second or rear portion **3503a**. In this particular embodiment, the first portion **3503b** has on one side thereof a button portion **3512**. It will be appreciated that column **3510**, adapter **3570**, collar **3502**, connector **3580**, and tubing **3505** can comprise biocompatible materials, such as PEEK or PEAK, PEKK, PEI, and/or PPSU, or can comprise other materials, such as stainless steel, nickel, steel, and/or titanium. As with the other embodiments described herein, the various items and features described and shown may comprise various materials which may be selected depending on the application to which the assembly **3501** is intended for, such as applications involving different pressures and/or different fluids of various pH levels, corrosivity, and the like.

Referring now to Fig. 35, the assembly **3501** is shown in a connected configuration in a cross-sectional view. As those skilled in the art will appreciate, the column **3510**, frit or filter **3562**, and holder **3560** with a tapered side **3561** may be like those shown and described elsewhere in this disclosure. As shown in Fig. 35, the adapter **3570** has at one end an internally threaded portion with threads adapted to engage with an externally threaded portion of one end of the column **3510**. A first end of the connector **3580** abuts against portions of the adapter **3570**. The connector **3580** includes a button portion **3512** on one side thereof, which is connected to a latching portion **3503b**. The first latching portion **3503b** is located within a recess on an exterior portion of the connector **3580** and also within a groove **3583** in an exterior portion of the adapter **3570**. In addition, the connection has a second latching portion **3503a**, which is located within a second groove in the exterior of the connector **3580**.

To disconnect the assembly **3501** from the connected configuration shown in Fig. 35, an operator can push on the button portion **3512**, thereby pushing the first latching portion **3503b** out of the grooves **3583** of the adapter **3570**, at which point the adapter **3570** and connector **3580** can be pulled apart from one another. From a disconnected configuration, the operator can connect the assembly **3501** by pushing the button portion **3512** radially inward and then inserting the adapter into the recess at one end of the connector **3580**, then releasing the button **3512** and allowing the first latching portion **3503b** to return to its location within the groove **3583** of the adapter **3570**, thereby retaining the adapter **3570** and the connector **3580** in a connected configuration.

As also shown in Fig. 35, the connector **3580** has a passageway therethrough which is adapted to receive and hold the tubing **3505**, as well as a spring **3520** proximal one end of the connector **3580**. The spring **3520** provides an axial loading force which biases the washer **3525** and therefore the sleeve **3510** and tubing **3505**, as well as the tip **3515**, towards the holder **3560**. The spring **3520** may be selected to provide a preselected force adapted to be sufficient to force the tip **3515** against one side of the holder **3560** as shown in Fig. 35 to provide a sealing engagement which does not leak and which will be sufficient to prevent the tubing **3505** from being forced out of a sealing engagement due to the intended pressures for the fluid flowing through the tubing **3505**. The holder **3560** has a tapered or frusto-conical portion **3561** to provide a reduced surface area against which the tip **3515** provides a sealing engagement. This allows the assembly **3501** to handle a greater fluid pressure without requiring an operator to significantly add a greater axial force to obtain a sealing engagement. As noted above, the tubing **3505**, holder **3560**, tip **3515**, filter **3562**, and/or the column **3510** may comprise biocompatible materials for applications in which biocompatibility is desired. In addition, these items may comprise materials selected for a particular intended application, such as those involving higher or lower pressures, or involving high or low pH levels, or those involving particular fluids, such as may be corrosive.

Those skilled in the art will appreciate that the embodiments shown and described in the present disclosure can be used in a variety of different configurations. For example, although the embodiments shown and described above have referred to use with flat-bottomed ports, those skilled in the art will appreciate that the assembly in its various embodiments of this disclosure can also be used in coned ports or other types of connections. In addition, various types of tubing and fitting assembly configurations may be used in addition to those shown and described, including without limitation those face-sealing assemblies shown and described in co-pending U.S. patent application Serial No. 14/922,041 filed on October 23, 2015, and titled "Face-Sealing Fluidic Connection System," which was published as U.S. published patent application US 2016/0116088 A1 on April 28, 2016, and is hereby incorporated by reference as fully set forth herein.

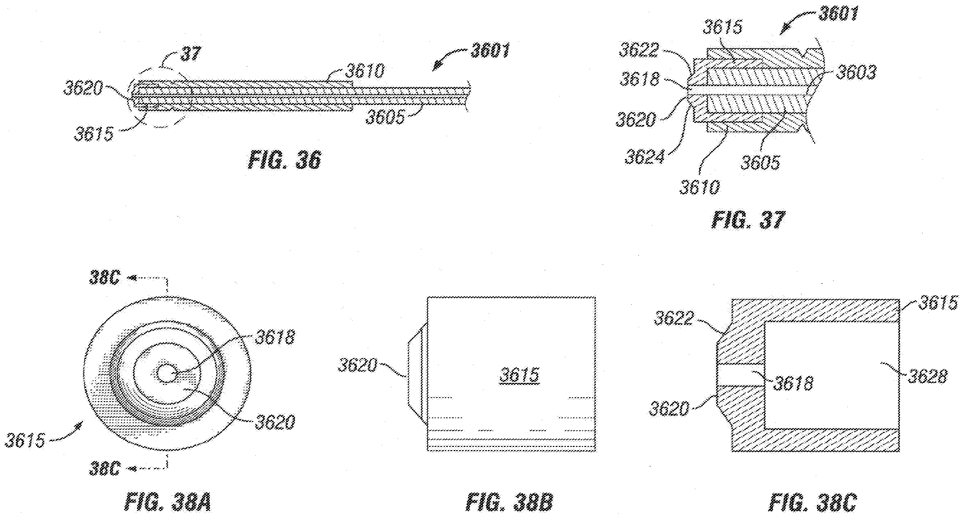

In addition to the tubing assemblies shown and described in US Published Patent Application No. 2016/0116088 A1, other types of tubing assemblies may be used in accordance with various embodiments of the present disclosure. Turning to Fig. 36, for example, a tubing assembly **3601** is shown in a partial cross section. The assembly **3601** includes a tube **3605**, a sleeve **3610**, and a tip **3615**. It can be seen from Fig. 36 that the tip **3615** has a first end with a reduced surface area **3620**. The reduced surface area **3620** extends outward and defines a first end of the tip **3615**, so that when the tubing assembly **3601** is inserted into a port or other connection assembly member to form a sealing connection, the reduced surface area **3620** is the portion of the tip **3615** which abuts the bottom of a port. By having reduced surface area **3620** in sealing engagement, the stresses are concentrated in a smaller area (i.e., the reduced surface area **3620** instead of over an area defined by the outer diameter of the tip **3615**), thus allowing for a sealing connection which can withstand higher pressures of fluid flow through the tubing assembly **3601**.

The tube **3605** can be any one of a number of materials. In one particular embodiment, tube **3604** may be a capillary tube. Similarly, sleeve **3610** can be comprised of various materials. Tip **3615** also can be made of various materials. The tubing **3605**, tip **3615**, and sleeve **3610** may comprise any one or more of the following materials, and need not comprise the same material as each other. Tubing **3605**, sleeve **3610**, and tip **3615** may comprise any one or more of materials, which may include metal, such as steel, including stainless steel, aluminum, titanium, as well as polymeric materials such as polyetheretherketone (PEEK), polyoxymethylene (POM) such as available under the mark DELRIN and known sometimes as acetal, RADEL brand polyphenylsulfone (PPSU), ULTEM brand polyetherimide (PEI), polyetherketoneketone (PEKK), polyaryletherketone (PAEK), polyethylene, polypropylene, polyvinylchloride, acrylic, and/or other materials, such a fused silica, silica borite, PEEKsil, and the like.

Referring now to Fig. 37, further details of the end of the tubing assembly **3601** can be seen. As shown in Fig. 37, one end of the tubing assembly **3601** may include an end of a capillary tube **3605** which has a passageway **3603** extending therethrough for fluid flow. The end of the tube **3605** fits within a seating portion of the tip **3615**. As shown in Fig. 37, tip **3615** has one end which covers and surrounds the outer surface of a portion of the tube **3605**. In addition, the tip **3615** has a second end which has a reduced surface area portion **3620**, and the second end of the tip **3615** is located between the end face of the tube **3605** and a port bottom surface (not shown in Fig. 37). As also shown in Fig. 37, the reduced surface area portion **3620** of the tip **3615** has a smaller diameter than the outer diameter of the tip **3615**, and extends outwardly from the shoulder portions **3622** and 3624 of the tip **3615**. Tip **3615** also has a passageway **3618** extending therethrough, which is preferably aligned with the fluid passageway **3603** of the tube **3605**.

Referring now to Figs. 38A, 38B, and 38C, additional views of the tip **3615** are provided. Fig. 38B is a side view of the tip **3615**, with the reduced surface area or boss **3620** shown on the left side. Fig. 38A provides a top view of the tip **3615**, and also shows the boss **3620** and the fluid pathway **3618**. Fig. 38C is a sectional view of the tip **3615** taken along line A - A of Fig. 38A, and shows the reduced surface area **3620**, the shoulder portion **3622** (which is shown as curved in Fig. 38C, but could be angled or even be provided with a right angle if desired). In addition, Fig. 38C shows the passageway **3618** through the end of the tip **3615** and also the hollow seating portion **3628**, which is adapted to receive the end of a tube therein, such as is shown in Fig. 37.

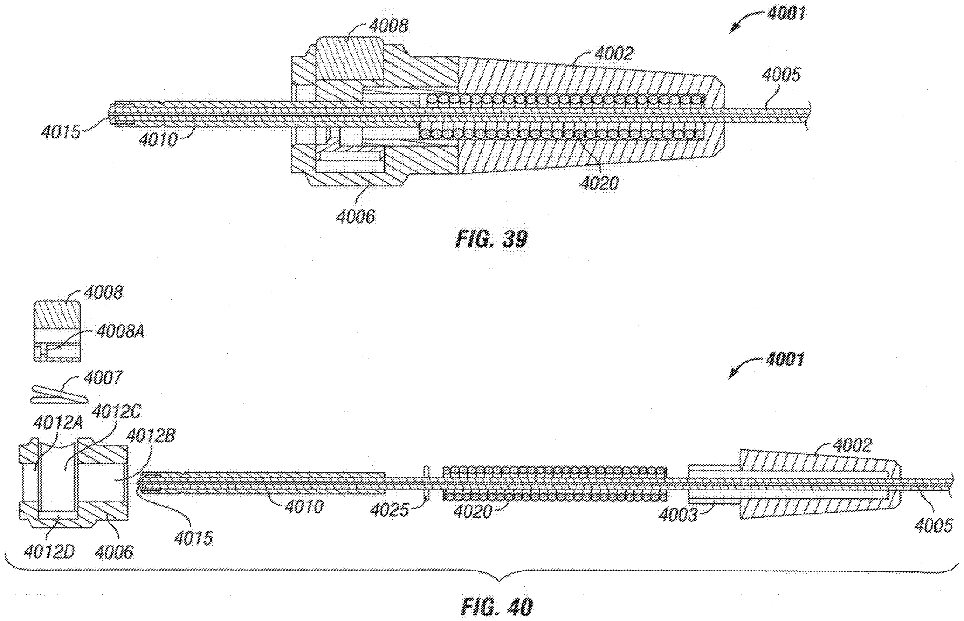

Referring now to Figs. 39 - 41, an alternative embodiment of a quick connect/disconnect assembly **4001** in accordance with the present disclosure is shown, together an embodiment of the quick connect/disconnect assembly **4001** with an adapter **4050** and a union **4070** to form a further assembly. In Fig. 39, the assembly **4001** is shown in a cross-sectional view in an assembled state. The assembly **4001** includes a body **4002**, tubing **4005**, a spring **4020**, a cap **4006**, and a latch **4008**. As shown in Fig. 39, the tubing **5** extends through each of the body **4002**, tubing **4005**, spring **4020**, cap **4006**, and latch **4008**, and extends from an opening on the left side of the assembly **4001**. At one of end of the tubing **4005**, a tip **4015** is provided. As also shown in Fig. 39, a portion of the tip **4015** and the tubing **4005** are enclosed by a sleeve **4010**. In addition, it can be seen that the spring **4020** is located within a hollow portion of the body **4002**, and one end of the spring **4020** abuts an interior surface of one end of the body **4002**. In addition, a second end of the body **4002** extends into an opening in the first end of the cap **4006** and into an opening in one end of the latch **4008**.

In Fig. 40, an exploded cross-sectional view of the assembly **4001** is provided. For convenience of the reader, like numerals are used in Figs. 39-41 for like features. As shown in Fig. 40, the body **4002** is generally hollow, with one end having an opening therethrough for the tubing **4005** and the other end having an opening adapted to receive the spring **4020**. The hollow portion of the body **4002** is adapted to receive and hold all or a portion of the spring **4020**. As shown in Figs. 39 - 41, the spring **4020** is a coiled spring, and can be made of a suitable metal. Those skilled in the art will appreciate, however, especially from the foregoing disclosure, that the spring **4020** may be any one of the various types of springs described above and made be made of any of the potential materials noted earlier with respect to the springs described above.

A washer **4025** is also shown in Fig. 40. The washer **4025** abuts against one end of the spring **4020**, while the other end of the spring **4020** abuts an interior surface of one end of the body **4002.** The relationship of the tube **4005**, sleeve **4010**, and tip **4015** can be as described above for such an assembly, and the tube **4005**, sleeve **4010,** and tip **4015** can be of any of the shapes and made of any of the materials noted above with respect to the earlier description thereof.

The cap **4006** shown in Fig. 40 has opposing openings **4012a** and **4012b** at opposing ends thereof. In addition, the cap **4006** has an opening **4012c** which extends into a hollow interior portion adapted to receive and hold the latch **4008.** In addition, the cap **4006** has a pocket **4012d** which is open to the hollow interior portion **4012c.** The pocket **4012d** is adapted to receive and hold a second spring **4007**. As shown in Fig. 40, the spring **4007** is a coiled tapered spring. Those skilled in the art will appreciate, however, that the spring **4007** may be any one of the types of springs described earlier in this disclosure.

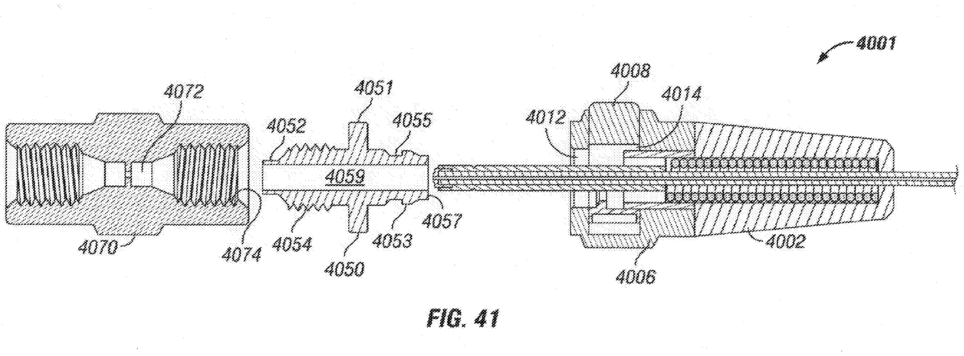

Still referring to Fig. 40, the latch **4008** is shown. The latch **4008** has a central passageway therethrough that is adapted to allow the tubing **4005,** sleeve **4010,** and tip **4015** combination (or just one or more components thereof) to fit through the latch **4008.** In addition, the latch **4008** includes a radially inwardly extending projection **4008a.** The radially inwardly projection **4008a** is adapted to cooperate with one end of an adapter **4050** like that shown in Fig. 41.

In Fig. 41, the assembly **4001** is shown with the tubing **4005** extending therethrough. In addition, an adapter **4050** and a union **4070** are shown. The adapter **4050** has a first end **4057** and a second end **4052**. The first end **4057** is adapted to fit into and be held securely by the openings **4012** in cap **4006** and the interior portion **4014** of the latch **4008.** As shown in Fig. 41, the first end **4057** has radially outward projections **4053,** as well as an annular notch or ring **4055** with a smaller outside diameter than the annular projection **4057**. In addition, the projection **4057** has a ramped portion facing towards the opening **4012**. The ramped portion is adapted to cooperate with a ramped portion of the projection **4008a** of the latch **4008**. The ramped portions are adapted so that no tools or excessive force is required to insert the first end **4057** of the adapter **4050** into the opening **4012** and engage the ramped portions so that the projection **4008a** fits into at least a portion of the annular notch **4055** and thereby securely holds the adapter **4050** and the cap **4006** (and thereby the assembly **4001)** together. In addition, the adapter **4050** includes a second annular flange which extends radially outwardly in a central portion of the adapter **4050.** Near the second end **4052** of the adapter **4050** are external threads **4054.**

As shown in Fig. 41, the external threaded portion **4054** of the adapter **4050** is adapted to removably engage with the internally threaded portion **4074** of a first end of the union **4070**. The first end of the union **4070** further has a flat-bottomed port **4072** for sealing engagement with the tip **4015** when the tip **4015** is pressed against the bottom of the port **4072**. Those skilled in the art will appreciate that, although a union **4070** is shown in Fig. 41, any one of a number of different components of an analytical instrument system may comprise a port that may be connected as described to the assembly **4001** and adapter **405** instead of the union **4070**. For example, other components which might have ports sealingly connected to the assembly **4001** and adapter **4050** instead of the union **4070** include at least the following: a column, or may be a port which is part of a manifold, a pump, a valve, a column, a filter, a guard column, a detector, a pressure regulator, a reservoir, a degasser, a debubbler, a union, a tee, a cross, a splitter, a sample loop, a connector, or another component in an AI instrument or system. The means for exerting an axial force may comprises a spring, including without limitation a coiled spring.

The fluidic connection assembly **4001,** as well as adapter **4050** and union **4070**, in accordance with the present disclosure may comprise any one or more of various materials, which may include metal, such as steel, including stainless steel, aluminum, titanium, as well as polymeric materials such as polyetheretherketone (PEEK), polyoxymethylene (POM) such as available under the mark DELRIN and known sometimes as acetal, RADEL brand polyphenylsulfone (PPSU), ULTEM brand polyetherimide (PEI), polyetherketoneketone (PEKK), polyaryletherketone (PAEK), polyethylene, polypropylene, polyvinylchloride, acrylic, and/or other materials, such a fused silica, silica borite, PEEKsil, and the like. The tubing **4005,** tip **4015,** and sleeve in the assembly **4001** similarly may comprise any one or more of the foregoing materials. In addition, those skilled in the art will appreciate that the various constituent components of the assembly **4001,** and/or adapter **4050** and/or union **4070**, may comprise the same or may comprise different materials. For example, assembly **4001** in one particular embodiment may have a body **4002** which comprises PEEK, a cap **4006** which comprises PEEK, and a latch **4008** which comprises PEEK, while springs **4020** and **4007** comprise a metal such as stainless steel, and such an assembly **4001** can be used with an adapter **4050** made of metal such as stainless steel, and a union **4070** which comprise a metal or PEEK. Those skilled in the art will further appreciate that, for those components made of polymeric materials, the polymeric material may include fibers, including carbon fibers, metallic fibers, and the like. It will be further appreciated that, even if an adapter **4050** comprises metal, the overall fluidic connection made with assembly **4001** can still be biocompatible, as long as the tubing tip **4015** of the tubing **4005** extending through the assembly **4001,** the adapter **4050,** and in sealing engagement with the bottom of the port **4072** of the union **4070,** as well as the union **4070,** comprise biocompatible materials.

Those skilled in the art will also appreciate that the material and size of spring members **4020** and **4007** can be selected so that the spring members **4020** and **4007** provide the desired amount of force, or biasing, when engaged (as described in more detail above) in a fluidic connection in a desired application. For example, one of skill would appreciate that it would be advantageous to have the spring member **4020** and spring **4007** provide a greater biasing force when the assembly **4001** will be used in an application with a greater fluidic pressure, than might be the case if the assembly **4001** is to be used in an application with a relatively small fluidic pressure. For example, not all fluidic connections in a given AI system used for a given application need operate under the same fluid pressure. The fluid pressures may change even in such situations for different connections in the AI system.

Methods of making a fluidic connection or disconnecting tubing from a port or component with respect to the assembly **4001** as shown in Figs. 39 - 41 are now summarized. In making a connection, an operator or user may perform the steps of providing a tube **4005** having first and second ends, wherein the second end may comprise a compressible tip, inserting one end of the tube through a body **4002,** wherein said body **4002** comprises a first end and a second end, said body **4002** further having a passageway therethrough adapted to receive a tube **4005** extending through said body, and wherein an interior portion proximal the second end of said body is adapted to cooperate with a spring **4020** for exerting a force against a washer or disc **4025** which in turn exerts a force against a sleeve **4010** which surrounds a portion of the tube **4005,** thus biasing the end of the tube **4005** in a longitudinal direction away from the body **4002.** In addition, the operator or user may insert a portion of a latch **4008** into a portion of a cap **4006** adapted to receive the latch **4008** portion. A spring **4007** may also be inserted into a recess in the cap **4006,** with the spring **4007** adapted to urge the latch upward and radially outwardly. The user or operator alternatively may simply obtain a tube **4005** and place one end through the interior passageway of the assembly **4001** once assembled. In either case, an adapter **4050** can be provided and the user or operator can insert one end of the tube **4005** into the passageway through the adapter **4050,** and insert one end **4057** of the adapter **4050** into the opening **4012** of the cap and the interior portions of latch **4008.** As noted, the annular projection or flange **4053** of the adapter has ramped portions adapted to engage with the ramped portions of the latch **4008a** to allow the user or operator to compress the spring **4020** more easily and have the latch **4008** securely engage with and hold the adapter **4050** in place. An operator or user can then insert the threaded end of the adapter **4050** into a port or other component such as any of the types described above, and then engage the threaded portion of the port or component (such as the union **4070)** to obtain a secure and sealed fluidic connection. To disconnect the assembly **4001** from the port or component, a user may turn the assembly **4001** and/or the port or other component (such as union **4070)** in the opposite direction to disengage the threaded portions from one another, then remove the adapter **4050** and assembly **4001** and the tube **4005** from the port or other component.

The spring **4020** may be selected to provide a preselected force or range of force adapted to be sufficient to force the tip **4015** against the bottom face of the port or component to provide a sealing engagement which does not leak and which will be sufficient to prevent the tubing **4005** from being forced out of a sealing engagement due to the intended pressures for the fluid flowing through the tubing **4005.** It will also be appreciated that the spring **4020** may be selected so that the force required to compress the spring, such as by inserting one end of the adapter **4050** into the cap **4006** and latch **4008** and securely engage the adapter **4050** with the assembly **4001,** is relatively limited and does not require any tools or equipment, but can be done by an operator or user by hand. Moreover, it will be appreciated that the adapter **4050** and assembly **4001** connection, once made, may be permanent or may be a removable connection, especially if the intended use of the assembly **4001** is for lower pressure applications and therefore the force required to keep the assembly **4001** and adapter **4050** secured together is not as great. In such situations, the second spring **4007** can be selected to provide a relatively lower force against the bottom of the latch **4008.**

Those skilled in the art will also appreciate that the embodiments shown and described in the present disclosure can be used in a variety of different configurations. For example, although the embodiments shown and described above have referred to use with flat-bottomed ports, those skilled in the art will appreciate that the assembly in its various embodiments of this disclosure can also be used in coned ports or other types of connections, including those described above. In addition, various types of tubing and fitting assembly configurations may be used in addition to those shown and described, including without limitation those face-sealing assemblies shown and described in co-pending U.S. patent application Serial No. 14/922,041 filed on October 23, 2015, and titled "Face-Sealing Fluidic Connection System," which was published as U.S. published patent application US 2016/0116088 A1 on April 28, 2016.

In addition to the tubing assemblies shown and described in US Published Patent Application No. 2016/0116088 A1, other types of tubing assemblies may be used in accordance with various embodiments of the present disclosure, such as those illustrated in Figs. 36 - 38 and described above, for example. The tube **4005** can be any one of a number of materials. In one particular embodiment, tube **4005** may be a capillary tube. Similarly, sleeve **4010** can be comprised of various materials. Tip **4015** also can be made of various materials. The tubing **4005,** tip **4015,** and sleeve **4010** may comprise any one or more of the following materials, and need not comprise the same material as each other, and such materials may include metal, such as steel, including stainless steel, aluminum, titanium, as well as polymeric materials such as polyetheretherketone (PEEK), polyoxymethylene (POM) such as available under the mark DELRIN and known sometimes as acetal, RADEL brand polyphenylsulfone (PPSU), ULTEM brand polyetherimide (PEI), polyetherketoneketone (PEKK), polyaryletherketone (PAEK), polyethylene, polypropylene, polyvinylchloride, acrylic, and/or other materials, such a fused silica, silica borite, PEEKsil, and the like.

We believe that the assembly **4001** can be used to achieve leak-free, sealing connections without any extrusion of tubing **4005** from a port (such as port **4072**) when fluid is flowing through the tubing **4005** at pressures of up to at least 20,000 psi, including for biocompatible applications.

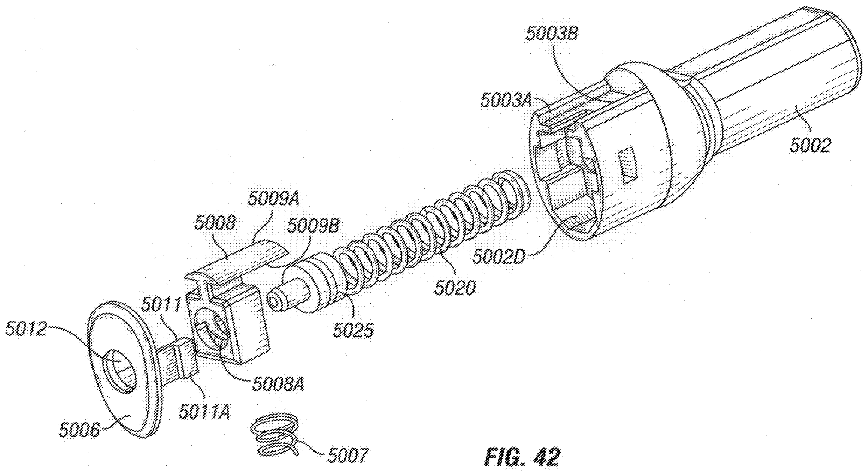

Referring now to Figs. 42 - 46, an alternative embodiment of the quick connect/disconnect assembly **5001** in accordance with the present invention is shown, together with an embodiment of the quick connect/disconnect assembly **5001** with an adapter **5050** to form the further assembly **5001a.** For convenience of the reader, like numerals are used in Figs. 42 - 46 for like features. In Fig. 42, the assembly **5001** is shown in an exploded three-dimensional view. The assembly **5001** includes a body **5002,** a spring **5020,** a cap **5006,** and a latch **5008.** Although not shown in Fig. 42, those skilled in the art will appreciate that tubing (not shown in Fig. 42) can extend through each of the body **5002,** spring **5020,** cap **5006,** and latch **5008,** and can extend from an opening on the left side of the assembly **5001,** as well as the right side of the assembly **5001**.

The body **5002** is generally hollow, with one end (the right hand side as shown in the orientation of Fig. 42) having an opening therethrough adapted to receive tubing (not shown in Fig. 42) and the other end (the left hand side in the orientation shown in Fig. 42) having an opening adapted to receive the spring **5020.** The hollow portion of the body **5002** is adapted to receive and hold all or a portion of the spring **5020.** As shown in Fig. 42, the spring **5020** is a coiled spring, and can be made of any suitable metal. Those skilled in the art will appreciate, however, especially from the foregoing disclosure, that the spring **5020** may be any one of the various types of springs described above and made be made of any of the potential materials noted earlier with respect to the springs described above. In addition, the body **5002** has a pocket **5002d** which is open to the body's hollow interior portion. The pocket **5002d** is adapted to receive and hold the second spring **4007**. As shown in Fig. 42, the spring **4007** is a coiled tapered spring. Those skilled in the art will appreciate, however, that the spring **4007** may be any one of the types of springs described earlier in this disclosure.

A washer **5025** is also shown in Fig. 42. The washer **5025** abuts against one end of the spring **5020,** while the other end of the spring **5020** abuts an interior surface of one end of the body **5002.** The cap **5006** shown in Fig. 42 has an opening **5012.** In addition, the cap **5006** has an extension **5011** on one side thereof, and the extension **5011** has a latching or holding member **5011a,** which projects radially inward towards the longitudinal axis of the assembly **5001.**

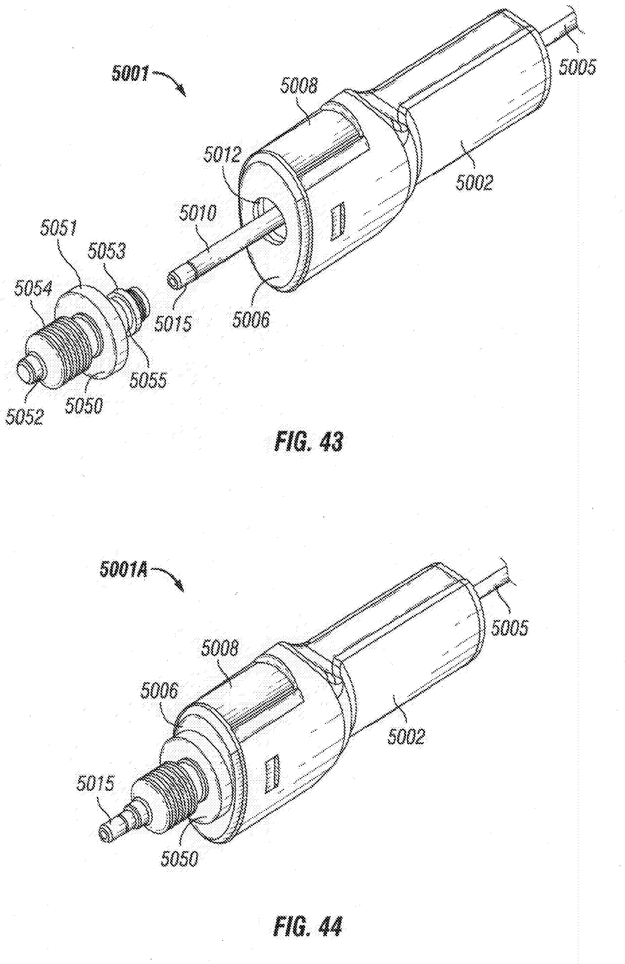

Still referring to Fig. 42, the latch **5008** is shown. The latch **5008** has a central passageway therethrough. In addition, the latch **5008** includes a radially inwardly extending projection **5008a.** The radially inwardly extending projection **5008a** is adapted to cooperate with one end of an adapter **4050** like that shown in Fig. 43, as explained in more detail below. The latch **5009** on its top portion in the orientation shown in Fig. 42 further has opposing projections **5009a** and **5009b**. As shown in Fig. 42, the top of the latch **5008** is angled or arcuate, so that the thickness of the latch **5008** at the outer sides of projections **5009a** and **5009b** is relatively thinner than towards the center of the top of the latch **5008.** It will be appreciated that the top of the latch **5008**, including projections **5009a** and **5009b,** is sized and adapted to slide into (and out of, if desired) the corresponding top right portion of the body **5002**. The body **5002** further includes recesses **5003a** and **5003b** which are adapted to removably receive and securely hold the projections **5009a** and **5009b,** respectively, of the latch **5008.** When assembled, the projections **5009a** and **5009b** fit into and are securely held in the recesses **5003a** and **5003b**.

Now referring to Fig. 43, the assembly **5001** is shown with tubing **5005** extending therethrough. In addition, an adapter **5050** is shown. The adapter **5050** has a first end and a second end. The first end of the adapter **5050** is adapted to fit into and be held securely in place by the interior extending projection **5008a** of the latch **5008**. As shown in Fig. 43, the first end has a radially outward projection **5053**, as well as an annular notch or ring **5055** with a smaller outside diameter than the annular projection **5053**. In addition, the projection **5053** has a ramped portion facing towards the opening **5012.** The ramped portion of the annular flange **5053** is adapted to cooperate with a ramped portion of the projection **5008a** of the latch **5008.** The ramped portions are adapted so that no tools or excessive force is required for an operator or user to insert the first end **5053** of the adapter **5050** into the opening **5012** and engage the ramped portions so that the projection **4008a** fits into at least a portion of the annular notch **5055** and thereby securely holds the adapter **5050** and the latch **5008** (and thereby the assembly **5001)** together. In addition, the adapter **5050** includes a second annular flange **5051** which extends radially outwardly in a central portion of the adapter **5050.** Near the second end **5052** of the adapter **5050** are external threads **5054.**

As also shown in Fig. 43, one end of the tubing **5005** has a tip **5015** provided. As also shown in Fig. 43, at least a portion of the tip **5015** and the tubing **5005** are enclosed by a sleeve **5010.** The relationship of the tube **5005,** sleeve **5010,** and tip **5015** can be as described above, and the tube **5005,** sleeve **5010,** and tip **5015** can be of any of the shapes and made of any of the materials noted above with respect to the earlier description thereof. Those skilled in the art will further appreciate that other types of tubing, such as without tip **5015** and/or without a sleeve **5010,** or with different configurations for either or both the tip **5015** and/or sleeve **5010,** can be used with the assembly **5001** as described herein.

In Fig. 44, assembly **5001a** is shown in an assembled state. The assembly **5001a** includes the body **5002,** tubing **5005,** the latch **5008,** the cap **5006,** the adapter **5050,** and the tip **5015.** As can be seen from a comparison of Figs. 43 and 44, Fig. 44 shows the adapter **5050** as inserted into the opening of the cap **5006** and held by the latch **5008** projection **5008a** (not seen in Fig. 44). As assembled, the adapter **5050,** cap **5006,** latch **5008,** and body **5002** form an assembly **5001a** which will securely hold together, and which can be used to secure one end of the tubing **5005** (or, as shown in Fig. 44, the tip **5015** at one end of the tubing **5005)** to a port of or to another component in a fluidic system, such as an analytical instrument system.

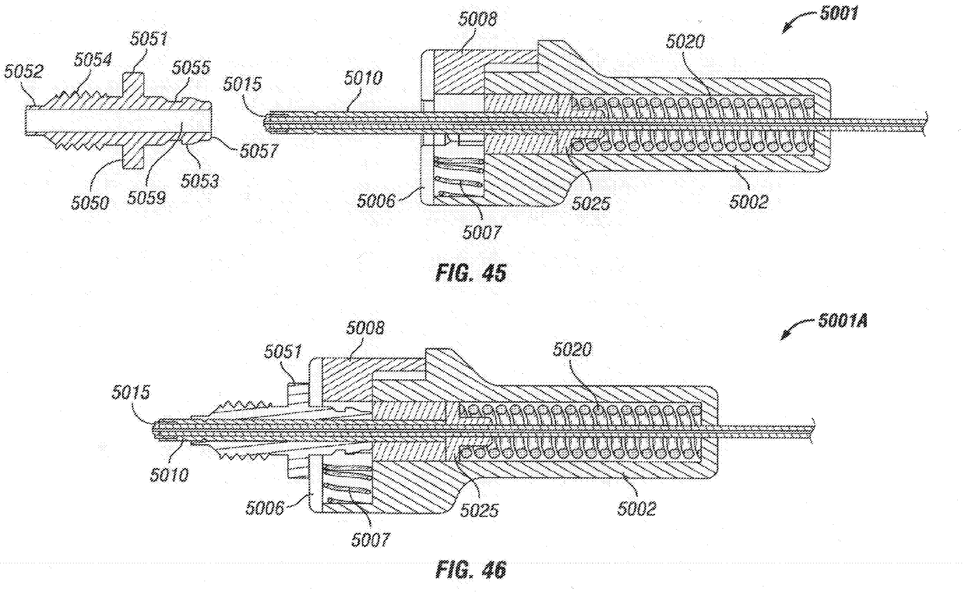

Referring now to Fig. 45, a cross-sectional view of the assembly **5001** and the adapter **5050** is provided. As can be seen, the adapter **5050** has a first end **5057** and a second end **5052.** Proximal the first end **5057** is an annular flange or radially outwardly extending projection **5053.** The ramped portion of the flange **5053** is shown and is located on the side of the flange **5053** facing the assembly **5001.** In addition, a portion of the adapter behind the flange **5053** has a smaller diameter and can be considered a notch portion **5055** directly behind or proximal behind the flange **5053.** In addition, the adapter has external threads **5054** and a second end adapted to fit into and form a seal in a flat bottomed port or other component.

Still referring to Fig. 45, it can be seen that the spring **5020** is located within a hollow portion of the body **5002,** and one end of the spring **5020** abuts an interior surface of one end of the body **5002**. In addition, a second end of the body **5002** is removably attached to one side of the latch **5008.** As shown in Fig. 45, the second end of the body **5002** provides a recess in which the second spring **5007** is located. As will be appreciated by those skilled in the art, the second spring **5007** is used to press the inward projection **5008a** of the latch **5008** into the notch **5055** of the adapter **5050** when the first end **5057** of the adapter **5050** is inserted into the second end of the assembly **5001**. Similarly, it will be appreciated that the spring **5020** is adapted to press the washer **5025** and, in turn, one end of the sleeve **5010** and also the tip **5015,** away from the second end of the body **5002** (and towards the left side of the view shown in Fig. 45 in this orientation of the assembly **5001).**

It will be appreciated that the external threaded portion **5054** of the adapter **5050** is adapted to removably engage with an internally threaded portion of a port or other component (not shown in Fig. 45). In the particular configuration of the adapter **5050** as shown, it will be appreciated the end **5052** of the adapter **5050** is adapted for use with a flat-bottomed port for sealing engagement with the tip **5015** when the tip **5015** is pressed against the bottom of the port. Those skilled in the art will appreciate that, although a flat-bottomed port has been described, any one of a number of different components of an analytical instrument system may comprise a port that may be connected as described to the assembly **5001** and adapter **5050**. For example, other components which might have ports sealingly connected to the assembly **5001** and adapter **5050** include at least the following: a column, or may be a port which is part of a manifold, a pump, a valve, a column, a filter, a guard column, a detector, a pressure regulator, a reservoir, a degasser, a debubbler, a union, a tee, a cross, a splitter, a sample loop, a connector, or another component in an AI instrument or system. The means for exerting an axial force may comprises a spring, including without limitation a coiled spring. In addition, those skilled in the art will appreciate that, although not shown, the end **5052** of the adapter **5050** may be of a configuration that is adapted to provide a sealing engagement in a frusto-conical shaped port or component.

Fig. 46 provides a cross-sectional view of the assembly **5001a** as assembled and ready for insertion into a flat-bottomed port or component of an analytical instrument system (not shown) to provide a sealing engagement.

It will be appreciated from the foregoing disclosure that the assembly **5001a** allows an operator or user to easily insert the end of the tubing having the tip **5015** and the end **5052** of the adapter **5050** into a flat-bottomed port by engaging internal threads of the port or component (not shown) with the external threads of the adapter **5050**. The force exerted by the biasing of the spring **5020** will maintain a sealing engagement of the tip **5015** and the bottom face of the port of component. We believe that the assembly **5001a** can be used to achieve leak-free, sealing connections without any extrusion of tubing **5005** from a port when fluid is flowing through the tubing **5005** at pressures of up to at least 20,000 psi, including for biocompatible applications.

The fluidic connection assembly **5001** and assembly **5001a,** as well as adapter **5050,** in accordance with the present disclosure may comprise any one or more of various materials, which may include metal, such as steel, including stainless steel, aluminum, titanium, as well as polymeric materials such as polyetheretherketone (PEEK), polyoxymethylene (POM) such as available under the mark DELRIN and known sometimes as acetal, RADEL brand polyphenylsulfone (PPSU), ULTEM brand polyetherimide (PEI), polyetherketoneketone (PEKK), polyaryletherketone (PAEK), polyethylene, polypropylene, polyvinylchloride, acrylic, and/or other materials, such a fused silica, silica borite, PEEKsil, and the like. The tubing **5005,** tip **5015,** and sleeve **5010** in the assembly **5001** similarly may comprise any one or more of the foregoing materials. In addition, those skilled in the art will appreciate that the various constituent components of the assembly **5001,** and/or adapter **5050,** may comprise the same or may comprise different materials. For example, assembly **5001** in one particular embodiment may have a body **5002** which comprises PEEK, a cap **5006** which comprises PEEK, and a latch **5008** which comprises PEEK, while springs **5020** and **5007** comprise a metal such as stainless steel, and such an assembly **5001** can be used with an adapter **5050** made of metal such as stainless steel. Those skilled in the art will further appreciate that, for those components made of polymeric materials, the polymeric material may include fibers, including carbon fibers, metallic fibers, and the like. It will be further appreciated that, even if an adapter **5050** comprises metal, the overall fluidic connection made with assembly **5001a** can still be biocompatible, as long as the tubing tip **5015** of the tubing **5005** extending through the assembly **5001a,** and in sealing engagement with the bottom of the port, comprise biocompatible materials.

Those skilled in the art will also appreciate that the material and size of spring members **5020** and **5007** can be selected so that the spring members **5020** and **5007** provide the desired amount of force, or biasing, when engaged (as described in more detail above) in a fluidic connection in a desired application. For example, one of skill would appreciate that it would be advantageous to have the spring member **5020** and spring **5007** provide a greater biasing force when the assembly **5001** will be used in an application with a greater fluidic pressure, than might be the case if the assembly **5001** is to be used in an application with a relatively small fluidic pressure. For example, not all fluidic connections in a given AI system used for a given application need operate under the same fluid pressure. The fluid pressures may change even in such situations for different connections in the AI system.

Methods of making a fluidic connection or disconnecting tubing from a port or component with respect to the assembly **5001** and Figs. 42 - 46 are now summarized. In making a connection, an operator or user may perform the steps of providing a tube **5005** having first and second ends, wherein the second end may comprise a compressible tip, inserting one end of the tube through a body **5002,** wherein said body **5002** comprises a first end and a second end, said body **5002** further having a passageway therethrough adapted to receive a tube **5005** extending through said body, and wherein an interior portion proximal the second end of said body is adapted to cooperate with a spring **5020** for exerting a force against a washer or disc **5025** which in turn exerts a force against a sleeve **5010** which surrounds a portion of the tube **5005,** thus biasing the end of the tube **5005** in a longitudinal direction away from the body **5002**. In addition, the operator or user may insert a portion (such as projections **5009a** and **5009b**) of a latch **5008** into a portion of the body **5002** adapted to receive and hold them, and insert a portion of a cap **5006** into or around an exterior portion of the latch **5008** (such as projection **5011a** and **5011**, which are adapted to engage with a portion of latch **5008** and hold cap **5006** and latch **5008** together). A spring **5007** may also be inserted into a recess in the body **5002**, with the spring **5007** adapted to urge the latch **5008** upward and radially outwardly.

The user or operator alternatively may simply obtain a tube **5005** and place one end through the interior passageway of the assembly **5001** once assembled. In either case, an adapter **5050** can be provided and the user or operator can insert one end of the tube **5005** into the passageway through the adapter **5050**, and insert one end **5057** of the adapter **5050** into the opening of the cap **5006** and the interior portions of latch **5008** and/or body **5002**. As noted and described above, the annular projection or flange **5053** of the adapter **5050** has ramped or angled portions adapted to engage with the ramped portions of the latch **5008** to allow the user or operator to compress the spring **5020** more easily and have the latch **5008** securely engage with and hold the adapter **5050** in place. An operator or user can then insert the threaded end of the adapter **5050** into a port or other component such as any of the types described above, and then engage the threaded portion of the port or component (not shown) to obtain a secure and sealed fluidic connection. To disconnect the assembly **5001** from the port or component, a user may turn the assembly **5001** and/or the port or other component (not shown) in the opposite direction to disengage the threaded portions from one another, then remove the adapter **5050** and assembly **5001** and the tube **5005** from the port or other component.

The spring **5020** may be selected to provide a preselected force or range of force adapted to be sufficient to force the tip **5015** against the bottom face of the port or component to provide a sealing engagement which does not leak and which will be sufficient to prevent the tubing **5005** from being forced out of a sealing engagement due to the intended pressures for the fluid flowing through the tubing **5005**. It will also be appreciated that the spring **5020** may be selected so that the force required to compress the spring, such as by inserting one end of the adapter **5050** into the cap **5006** and latch **5008** and securely engage the adapter **5050** with the assembly **5001**, is relatively limited and does not require any tools or equipment, but can be done by an operator or user by hand. Moreover, it will be appreciated that the adapter **5050** and assembly **5001** connection, once made, may be permanent or may be a removable connection, especially if the intended use of the assembly **5001** is for lower pressure applications and therefore the force required to keep the assembly **5001** and adapter **5050** secured together is not as great. In such situations, the second spring **5007** can be selected to provide a relatively lower force against the bottom of the latch **5008**.

Those skilled in the art will also appreciate that the embodiments shown and described in the present disclosure can be used in a variety of different configurations. For example, although the embodiments shown and described above have referred to use with flat-bottomed ports, those skilled in the art will appreciate that the assembly in its various embodiments of this disclosure can also be used in coned ports or other types of connections, including those described above. In addition, various types of tubing and fitting assembly configurations may be used in addition to those shown and described, including without limitation those face-sealing assemblies shown and described in co-pending U.S. patent application Serial No. 14/922,041 filed on October 23, 2015, and titled "Face-Sealing Fluidic Connection System," which was published as U.S. published patent application US 2016/0116088 A1 on April 28, 2016.

In addition to the tubing assemblies shown and described in US Published Patent Application No. 2016/0116088 A1, other types of tubing assemblies may be used in accordance with various embodiments of the present disclosure, such as those illustrated in Figs. 42 - 46 and described above, for example. The tube **5005** can be any one of a number of materials. In one particular embodiment, tube **5005** may be a capillary tube. Similarly, sleeve **5010** can be comprised of various materials. Tip **5015** also can be made of various materials. The tubing **5005,** tip **5015,** and sleeve **5010** may comprise any one or more of the following materials, and need not comprise the same material as each other, and such materials may include metal, such as steel, including stainless steel, aluminum, titanium, as well as polymeric materials such as polyetheretherketone (PEEK), polyoxymethylene (POM) such as available under the mark DELRIN and known sometimes as acetal, RADEL brand polyphenylsulfone (PPSU), ULTEM brand polyetherimide (PEI), polyetherketoneketone (PEKK), polyaryletherketone (PAEK), polyethylene, polypropylene, polyvinylchloride, acrylic, and/or other materials, such a fused silica, silica borite, PEEKsil, and the like.

Those skilled in the art will appreciate that various materials can be used in place of or in addition to those described herein, and that the embodiments shown and described can be used in additional applications and provide additional advantages beyond those set forth herein. Hence, the embodiments shown and described in the drawings and the above discussion are merely illustrative and do not limit the scope of the invention as defined in the claims herein. The embodiments and specific forms, materials, and the like are merely illustrative and do not limit the scope of the invention or the claims herein.

## Claims

1. A method of connecting a tube (5005) to a port, comprising:
providing a tube (5005) having first and second ends, wherein the second end comprises a tip (5015);
inserting the first end of the tube through a first spring (5020) and a body (5002), wherein said body (5002) comprises a first end, a second end, and a passageway therethrough adapted to receive the tube (5005) extending through said body (5002);
inserting at least a portion of a latch member (5008) having an opening, into a portion of said body (5002), wherein one or more projections (5009a) of the latch member (5008) are adapted to be held in one or more recesses (5003a, 5003b) on said portion of said body (5002);
inserting a portion (5011, 5011a) of a cap member (5006) having an opening (5012), into a portion of the latch member (5008), wherein said portion (5011, 5011a) of the cap (5006) is adapted to hold the cap member (5006) and latch member (5008) together; and
inserting at least a portion of the body (5002), including at least the latch member (5008), into a port, wherein the latch member (5008) is retained within the port.

2. The method according to claim 1 wherein the step of inserting at least said portion of the body (5002) into said port does not require any torque; or wherein the step of inserting at least said portion of the body (5002) into said port does not require a force of more than about 11.1N (2.5 pounds), and does not require the application of a torque; or wherein the step of inserting at least said portion of the body (5002) into said port does not require a force of more than about 44.5N (10 pounds), and does not require the application of a torque; or wherein the step of inserting at least said portion of the body (5002) into said port does not require a force of more than about 40.0N (9 pounds), and does not require the application of a torque; or wherein the step of inserting at least said portion of the body (5002) into said port does not require a force of more than about 35.6N (8 pounds), and does not require the application of a torque.

3. A fluidic connection assembly (5001) comprising:
a body (5002) having a first end and a second end, each of the first and second ends having openings therein, and having a hollow portion therein;
a first spring (5020) located at least partially within the hollow portion of said body (5002), and having a first end abutting an interior surface of the hollow portion of said body (5002);
a latch member (5008) having an opening therethrough and having a base portion and a top portion, wherein the base portion of said latch member (5008) is adapted to fit at least partially within said body (5002), and wherein the top portion has one or more projections (5009a) adapted to be held in recesses (5003a, 5003b) on an exterior portion of said body (5002);
a cap member (5006) having an opening (5012) therethrough and having first and second sides and an extension (5011, 5011a) on the first side of said cap member (5006) which is adapted to extend into at least a portion of the hollow portion of said body (5002) and to hold said latch member (5008) and said cap member (5006) together, wherein the second side of said cap member (5006) is adapted to be attached to the second end of said body (5002); and
wherein said latch member (5008) is adapted to engage with and securely hold an adapter (5050) when a portion of one end of the adapter (5050) is inserted into the opening of said cap member (5006) and the opening of said latch member (5008), and wherein each of said body (5002), said spring (5020), said latch member (5008), and said cap member (5006) are adapted to receive at least a portion of tubing (5005) therethrough.

4. The fluidic connection assembly (5001) of claim 3 wherein said first spring (5020) is in a compressed state when said cap member (5006), said latch member (5008), and said body (5002) are assembled together and said first spring (5020) is located entirely within the hollow portion of said body (5002); or
further comprising a second spring (5007), wherein said second spring (5007) is located within the hollow portion of said body (5002) and adjacent to at least one side of said latch member (5008), wherein said second spring (5007) is optionally adapted to bias said latch member (5008) so that a radially inward projection of said latch member (5008) is pressed against a portion of said adapter (5050) when a first end of the adapter (5050) is inserted into said latch member (5008).

5. The fluidic connection assembly (5001) of claim 3 further comprising a tube (5005) having one end which extends through the first and second ends of said body (5002), said first spring (5020), said latch member (5008), and extends out of the opening of the second side of said cap member (5060), wherein said first spring (5020) optionally exerts a force which urges the end of said tube away from said body (5002); and optionally further comprising a washer (5025) located within said body (5002) and adjacent to a second end of said first spring (5020), a sleeve member (5010) surrounding at least a portion of said tube (5005), wherein said sleeve member (5010) has a first end and a second end, and wherein said first spring (5020) exerts a force on said washer (5025) and said washer (5025) exerts a force on said sleeve (5010), and said sleeve (5010) exerts a force on said tube (5005) which urges the end of said tube (5005) away from said body (5002).

6. The method according to claim 1 further comprising:
providing an adapter (5050); and
inserting one end of the adapter (5050) into the opening (5012) of the cap member (5006) and the opening of the latch member (5008), wherein each of said body (5002), the first spring (5020), the latch member (5008), and the cap member (5006) are adapted to receive at least a portion of the tube (5005) therethrough.

7. The method according to claim 1 further comprising:
inserting a second spring (5007) into a recess in said body (5002), wherein the second spring (5007) is located within said body (5002) and adjacent to at least one side of the latch member (5008), wherein the second spring (5007) is adapted to bias the latch member (5008) so that a radially inward projection (5008a) of the latch member (5008) is pressed against a portion of the adapter (5050) when the adapter (5050) is inserted into the latch member (5008).

8. The method according to claim 1 wherein an interior portion proximal to the second end of said body (5002) is adapted to cooperate with the first spring (5020) for exerting a force against a washer (5025).

9. The method according to claim 8 wherein the first spring (5020) is located at least partially within said body (5002) and the washer (5025) is located within said body (5002) and adjacent to one end of the first spring (5020).

10. The method according to claim 8 wherein the washer (5025) exerts a force against a sleeve (5010) surrounding at least a portion of the tube (5005), thereby biasing the end of the tube (5005) away from said body (5002).

11. The fluidic connection assembly (5001) of claim 3 wherein at least one of said body (5002), said latch member (5008), and said cap member (5006) comprise polyetherketoneketone.

12. The fluidic connection assembly (5001) of claim 3 wherein said assembly is adapted to provide a leak-free connection of one end of tubing when fluid is flowing through the tubing at pressures up to at least 5,000 psi.

13. The fluidic connection assembly (5001) of claim 3 wherein when a first end of said adapter (5050) is located within the second end of said body (5002), a radially inward projection (5008a) of said latch member (5008) presses against a portion of the first end of said adapter (5050).

14. The fluidic connection assembly (5001) of claim 13 wherein a second end of said adapter (5050) is adapted to removably engage a port.

15. The fluidic connection assembly (5001) of claim 14 wherein the port comprises a flat-bottomed port.

## Patentansprüche

1. Verfahren zum Verbinden eines Rohrs (5005) mit einem Anschluss, umfassend:
das Bereitstellen eines Rohrs (5005), das ein erstes und ein zweites Ende aufweist, wobei an dem zweiten Ende eine Spitze (5015) vorgesehen ist;
das Einführen des ersten Endes des Rohrs durch eine erste Feder (5020) und durch einen Körper (5002), wobei der Körper (5002) ein erstes Ende, ein zweites Ende und einen Durchgang aufweist, der so angepasst ist, dass er das sich durch den Körper (5002) erstreckende Rohr (5005) aufnehmen kann;
das Einsetzen von mindestens einem Teil eines mit einer Öffnung versehenen Verriegelungselements (5008) in einen Abschnitt des Körpers (5002), wobei ein oder mehrere Vorsprünge (5009a) des Verriegelungselements (5008) so angepasst sind, dass sie in einer oder in mehreren Aussparungen (5003a, 5003b) des Abschnitts des Körpers (5002) gehalten werden;
das Einsetzen eines Abschnitts (5011, 5011a) eines mit einer Öffnung (5012) versehenen Kappenelements (5006) in einen Bereich des Verriegelungselements (5008), wobei der Abschnitt (5011, 5011a) der Kappe (5006) dazu ausgelegt ist, das Kappenelement (5006) und das Verriegelungselement (5008) zusammenzuhalten; und
das Einführen von mindestens einem Teil des Körpers (5002), der mindestens das Verriegelungselements (5008) enthält, in einen Anschluss, wobei das Verriegelungselement (5008) in dem Anschluss gehalten wird.

2. Verfahren nach Anspruch 1, bei dem der Schritt des Einführens von zumindest dieses Teils des Körpers (5002) in den Anschluss kein Drehmoment erfordert; oder bei dem der Schritt des Einführens von zumindest dieses Teils des Körpers (5002) in den Anschluss keine Kraft von mehr als etwa 11,1N (2,5 brit. pounds) erfordert und kein Aufbringen eines Drehmoments erfordert; oder bei dem der Schritt des Einführens zumindest dieses Teils des Körpers (5002) in den Anschluss keine Kraft von mehr als etwa 44. 5N (10 brit. pounds) erfordert und nicht das Aufbringen eines Drehmoments erfordert; oder wobei der Schritt des Einführens von zumindest dieses Teils des Körpers (5002) in den Anschluss keine Kraft von mehr als etwa 40,0N (9 brit. pounds) erfordert und nicht das Aufbringen eines Drehmoments erfordert; oder wobei der Schritt des Einführens von zumindest dieses Teils des Körpers (5002) in den Anschluss keine Kraft von mehr als etwa 35,6N (8 brit. pounds) erfordert und nicht das Aufbringen eines Drehmoments erfordert.

3. Fluidische Verbindungsanordnung (5001), umfassend:
einen Körper (5002) mit einem ersten Ende und einem zweiten Ende, wobei sowohl das erste als auch das zweite Ende mit Öffnungen und mit einem hohlen Abschnitt darin versehen ist;
eine erste Feder (5020), die zumindest teilweise innerhalb des hohlen Abschnitts des Körpers (5002) angeordnet ist und ein erstes Ende aufweist, das an einer Innenfläche des hohlen Abschnitts des Körpers (5002) anliegt;
ein Verriegelungselement (5008) mit einer durchgehenden Öffnung und mit einem Basisabschnitt sowie einem oberen Abschnitt, wobei der Basisabschnitt des Verriegelungselements (5008) so angepasst ist, dass er zumindest teilweise in den Körper (5002) passt, und wobei der obere Abschnitt einen oder mehrere Vorsprünge (5009a) aufweist, die so angepasst sind, dass sie in Aussparungen (5003a, 5003b) an einem äußeren Abschnitt des Körpers (5002) gehalten werden;
ein Kappenelement (5006) mit einer durchgehenden Öffnung (5012) und mit einer ersten und einer zweiten Seite sowie einem Fortsatz (5011, 5011a) auf der ersten Seite des Kappenelements (5006), der so angepasst ist, dass er sich in mindestens einen Teil des hohlen Abschnitts des Körpers (5002) erstreckt und das Verriegelungselement (5008) und das Kappenelement (5006) zusammenzuhält, wobei die zweite Seite des Kappenelements (5006) so angepasst ist, dass sie an dem zweiten Ende des Körpers (5002) befestigt werden kann; und
wobei das Verriegelungselement (5008) so angepasst ist, dass es in einen Adapter (5050) eingreift und in diesem sicher gehalten wird, wenn ein Teil eines Endes des Adapters (5050) in die Öffnung des Kappenelements (5006) und in die Öffnung des Verriegelungselements (5008) eingesetzt wird, und wobei sowohl der Körper (5002) als auch die Feder (5020), das Verriegelungselement (5008) und das Kappenelement (5006) so angepasst sind, dass sie mindestens einen Teil eines Rohrs (5005) aufnehmen.

4. Fluidische Verbindungsanordnung (5001) nach Anspruch 3, wobei sich die erste Feder (5020) in einem zusammengedrückten Zustand befindet, wenn das Kappenelement (5006), das Verriegelungselement (5008) und der Körper (5002) zusammengefügt sind und sich die erste Feder (5020) vollständig innerhalb des hohlen Abschnitts des Körpers (5002) befindet; oder
ferner umfassend eine zweite Feder (5007), wobei die zweite Feder (5007) innerhalb des hohlen Abschnitts des Körpers (5002) und angrenzend an mindestens eine Seite des Verriegelungselements (5008) angeordnet ist, wobei die zweite Feder (5007) optional angepasst ist, um das Verriegelungselement (5008) so vorzuspannen, dass ein radial nach innen gerichteter Vorsprung des Verriegelungselements (5008) gegen einen Abschnitt des Adapters (5050) gedrückt wird, wenn ein erstes Ende des Adapters (5050) in das Verriegelungselement (5008) eingesetzt ist.

5. Fluidische Verbindungsanordnung (5001) nach Anspruch 3, ferner umfassend ein Rohr (5005), bei dem sich ein Ende durch das erste und das zweite Ende des Körpers (5002), durch die erste Feder (5020) und durch das Verriegelungselement (5008) erstreckt und aus der Öffnung der zweiten Seite des Kappenelements (5006) herausragt, wobei die erste Feder (5020) optional eine Kraft ausübt, die das Ende des Rohrs von dem Körper (5002) wegdrückt; und ferner optional umfassend eine Unterlegscheibe (5025), die innerhalb des Körpers (5002) und benachbart zu einem zweiten Ende der ersten Feder (5020) angeordnet ist, sowie ein Hülsenelement (5010), das zumindest einen Teil des Rohrs (5005) umgibt, wobei das Hülsenelement (5010) ein erstes Ende und ein zweites Ende aufweist, und wobei die erste Feder (5020) eine Kraft auf die Unterlegscheibe (5025) ausübt und die Unterlegscheibe (5025) eine Kraft auf die Hülse (5010) ausübt und wobei die Hülse (5010) eine Kraft auf das Rohr (5005) ausübt, die das Ende des Rohrs (5005) von dem Körper (5002) wegdrückt.

6. Verfahren nach Anspruch 1 ferner umfassend:
das Bereitstellen eines Adapters (5050); und
das Einsetzen eines Endes des Adapters (5050) in die Öffnung (5012) des Kappenelements (5006) und in die Öffnung des Verriegelungselements (5008), wobei sowohl der Körper (5002) als die erste Feder (5020), das Verriegelungselement (5008) und das Kappenelement (5006) so angepasst sind, dass sie zumindest einen Teil des Rohrs (5005) aufnehmen.

7. Verfahren nach Anspruch 1, ferner umfassend:
das Einsetzen einer zweiten Feder (5007) in eine in dem Körper (5002) vorgesehene Aussparung, wobei die zweite Feder (5007) innerhalb des Körpers (5002) und angrenzend an mindestens eine Seite des Verriegelungselements (5008) angeordnet ist, wobei die zweite Feder (5007) dazu angepasst ist, das Verriegelungselement (5008) so vorzuspannen, dass ein radial nach innen gerichteter Vorsprung (5008a) des Verriegelungselements (5008) gegen einen Abschnitt des Adapters (5050) gedrückt wird, wenn der Adapter (5050) in das Verriegelungselement (5008) eingesetzt wird.

8. Verfahren nach Anspruch 1, wobei ein innerer Abschnitt in der Nähe des zweiten Endes des Körpers (5002) so angepasst ist, dass er mit der ersten Feder (5020) zusammenwirkt, um eine Kraft gegen eine Unterlegscheibe (5025) auszuüben.

9. Verfahren nach Anspruch 8, wobei die erste Feder (5020) zumindest teilweise innerhalb des Körpers (5002) angeordnet ist und die Unterlegscheibe (5025) innerhalb des Körpers (5002) und benachbart zu einem Ende der ersten Feder (5020) angeordnet ist.

10. Verfahren nach Anspruch 8, bei dem die Unterlegscheibe (5025) eine Kraft gegen eine Hülse (5010) ausübt, die mindestens einen Teil des Rohrs (5005) umgibt, wodurch das Ende des Rohrs (5005) von dem Körper (5002) weggedrückt wird.

11. Fluidische Verbindungsanordnung (5001) nach Anspruch 3, wobei mindestens eines von den folgenden Teilen, dem Körper (5002), dem Verriegelungselement (5008) und dem Kappenelement (5006), aus Polyetherketonketon besteht.

12. Fluidische Verbindungsanordnung (5001) nach Anspruch 3, wobei die Anordnung so angepasst ist, dass sie eine leckagefreie Verbindung eines Endes der Rohrleitung bereitstellt, wenn Fluid bei einem Druck von bis zu mindestens 5.000 psi durch die Rohrleitung strömt.

13. Fluidische Verbindungsanordnung (5001) nach Anspruch 3, wobei, wenn ein erstes Ende des Adapters (5050) innerhalb des zweiten Endes des Körpers (5002) angeordnet ist, ein radial nach innen gerichteter Vorsprung (5008a) des Verriegelungselements (5008) gegen einen Teil des ersten Endes des Adapters (5050) drückt.

14. Fluidische Verbindungsanordnung (5001) nach Anspruch 13, bei der ein zweites Ende des Adapters (5050) so angepasst ist, dass es lösbar in eine Öffnung eingreift.

15. Fluidische Verbindungsanordnung (5001) nach Anspruch 14, bei der die Öffnung einen Anschluss mit flachem Boden aufweist.

## Revendications

1. Procédé de connexion d'un tube (5005) à un orifice consistant à :
- fournir un tube (5005) ayant une première et une seconde extrémité, la seconde extrémité ayant un embout (5015),
- introduire la première extrémité du tube à travers un premier ressort (5020) et un corps (5002), le corps (5002) ayant une première extrémité, une seconde extrémité et un passage traversant pour recevoir le tube (5005) s'étendant à travers le corps (5002),
- insérer au moins une partie d'un élément de verrou (5008) ayant un orifice dans une partie du corps (5002), une ou plusieurs projections (5009a) du verrou (5008) étant adaptées pour être tenues dans une ou plusieurs cavités (5003a, 5003b) de la partie de corps (5002),
- insérer une partie (5011, 5011a) d'un capuchon (5006) ayant une ouverture (5012) dans une partie du verrou (5008), cette partie (5011, 5011a) du capuchon (5006) étant adaptée pour tenir le capuchon (5006) et le verrou (5008) l'un à l'autre, et
- insérer au moins une partie du corps (5002) comprenant au moins le verrou (5008) dans un orifice, le verrou (5008) étant retenu dans l'orifice.

2. Procédé selon la revendication 1,
selon lequel
l'étape d'insertion d'au moins une partie du corps (5002) dans l'orifice ne nécessite pas l'application d'un couple ou encore l'étape d'insertion au moins une partie du corps (5002) dans l'orifice ne nécessite pas de force supérieure à 11,1N (2,5 livres) et ne nécessite pas l'application d'un couple ; ou encore l'étape d'insertion d'au moins une partie du corps (5002) dans l'orifice ne nécessite pas une force supérieure à environ 44,5 N (10 livres) et ne nécessite pas l'application d'un couple ; ou encore l'étape d'insertion d'au moins une partie du corps (5002) dans l'orifice ne nécessite pas une force supérieure à environ 40,0 N (9 livres) et ne nécessite pas l'application d'un couple ; ou encore l'étape d'insertion d'au moins une partie du corps (5002) dans l'orifice ne nécessite pas une force supérieure à environ 35,6 N (8 livres) et ne nécessite pas l'application d'un couple.

3. Ensemble de connexions fluidique (5001) comprenant :
un corps (5002) ayant une première extrémité et une seconde extrémité,
chacune des deux extrémités ayant des orifices et une partie en creux,
- un premier ressort (5020) étant situé au moins en partie dans le creux du corps (5002) et ayant une première extrémité venant contre la surface intérieure de la partie en creux du corps (5002),
- un verrou (5008) ayant un orifice traversant et une partie de base ainsi qu'une partie de dessus, la partie de base du verrou (5008) étant adaptée pour se loger au moins partiellement dans le corps (5002) et la partie du dessus ayant une ou plusieurs projections (5009a) adaptées pour être tenues dans des cavités (5003a, 5003b) sur la partie extérieure du corps (5002),
- un capuchon (5006) ayant un orifice (5012) traversant et un premier et un second côtés ainsi qu'un prolongement (5011, 5011a) sur le premier côté du capuchon (5006) qui est adapté pour s'étendre dans au moins une partie du creux du corps (5002) et tenir le verrou (5008) et le capuchon (5006) ensemble, le second côté du capuchon (5006) étant adapté pour être fixé à la seconde extrémité du corps (5002), et
le verrou (5008) est adapté pour coopérer et tenir fermement un adapteur (5050) lorsqu'une partie d'une extrémité de l'adaptateur (5050) est insérée dans l'orifice du capuchon (5006) et l'ouverture du verrou (5008) à la fois le corps (5002) et le ressort (5020), le verrou (5008) et le capuchon (5006) sont adaptés pour recevoir au moins une partie d'un tube (5005).

4. Ensemble de connexions fluidique (5001) selon la revendication 3, dans lequel
le premier ressort (5020) est à l'état comprimé lorsque le capuchon (5006), le verrou (5008) et le corps (5002) sont assemblés et le premier ressort (5020) est situé complètement dans le creux du corps (5002), ou comprenant en outre un second ressort (5007), le second ressort (5007) étant situé dans la partie creuse du corps (5002) et de façon adjacente à au moins un côté du verrou (5008), le second ressort (5007) étant en option adapté pour pousser le verrou (5008) pour que la partie radialement vers l'intérieur du verrou (5008) soit pressée contre une partie de l'adaptateur (5050) lorsque la première extrémité de l'adaptateur (5050) est introduite dans le verrou (5008).

5. Ensemble de connexions fluidique (5001) selon la revendication 3, comprenant en outre un tube (5005) ayant une première extrémité qui traverse la première et la seconde extrémités du corps (5002), le premier ressort (5020), le verrou (5008) et sort de l'orifice du second côté du capuchon (5060), le premier ressort (5020) exerçant en option une force qui pousse l'extrémité du tube pour l'écarter du corps (5002) et en option, il comprend en outre une rondelle (5025) près du corps (5002) et adjacente à la seconde extrémité du premier ressort (5020), un manchon (5010) entourant au moins en partie le tube (5005), le manchon (5010) ayant une première extrémité et une seconde extrémité et le premier ressort (5020) exerce une force sur la rondelle (5025) et cette rondelle (5025) exerce une force sur le manchon (5010), le manchon (5010) exerçant une force sur le tube (5005) qui pousse l'extrémité du tube (5005) pour l'écarter du corps (5002).

6. Procédé selon la revendication 1,
consistant en outre à :
- fournir un adaptateur (5050), et
- insérer une extrémité de l'adaptateur (5050) dans l'orifice (5012) du capuchon (5006) et l'ouverture du verrou (5008), le corps (5002), le premier ressort (5020), le verrou (5008) et le capuchon (5006) étant adaptés chacun pour recevoir au moins une partie du tube (5005) à travers eux.

7. Procédé selon la revendication 1,
consistant en outre à :
- insérer un second ressort (5007) dans la cavité du corps (5002), le second ressort (5007) étant situé dans le corps (5002) adjacent à au moins un côté du verrou (5008), le second ressort (5007) étant adapté pour pousser le verrou (5008) de façon que la projection radialement intérieure (5008a) du verrou (5008) soit pressée contre une partie de l'adaptateur (5050) lorsque l'adaptateur (5050) est inséré dans le verrou (5008).

8. Procédé selon la revendication 1,
selon lequel
la partie intérieure proche de la seconde extrémité du corps (5002) est adaptée pour coopérer avec le premier ressort (5020) pour exercer une force contre une rondelle (5025).

9. Procédé selon la revendication 8,
selon lequel
le premier ressort (5020) est situé au moins en partie dans le corps (5002) et la rondelle (5025) est située dans le corps (5002) et elle est adjacente à une extrémité du premier ressort (5020).

10. Procédé selon la revendication 8,
selon lequel
la rondelle (5025) exerce une force contre un manchon (5010) entourant au moins une partie du tube (5005) de façon à pousser l'extrémité du tube (5005) pour l'écarter du corps (5002).

11. Assemblage de connexions fluidiques (5001) selon la revendication 3,
selon lequel
au moins le corps (5002), le verrou (5008), le capuchon (5006) sont chacun en polyéther-cétone-cétone.

12. Assemblage de connexions fluidiques (5001) selon la revendication 3,
selon lequel
l'ensemble est adapté pour réaliser un branchement sans fuite d'une extrémité d'un tube lorsque du liquide traverse le tube à des pressions allant au moins jusqu'à 5000 psi.

13. Assemblage de connexions fluidiques (5001) selon la revendication 3,
selon lequel la première extrémité d'adaptateur (5050) est située dans la seconde extrémité du corps (5002), une projection radialement vers l'intérieur (5008a) du verrou (5008) pressant contre une partie de la première extrémité de l'adaptateur (5050).

14. Ensemble de connexions fluidiques (5001) selon la revendication 13,
selon lequel
la seconde extrémité de l'adaptateur (5050) est adaptée pour s'engager de manière amovible dans un orifice.

15. Ensemble de connexions fluidiques (5001) selon la revendication 14,
selon lequel
l'orifice comporte un orifice à fond plat.
